# EUROPÄISCHE PATENTSCHRIFT

(11) **EP 1 891 220 B1**
(45) Veröffentlichungstag und Bekanntmachung des Hinweises auf die Patenterteilung: **31.03.2010**
(21) Anmeldenummer: 06753229.1
(22) Anmeldetag: 02.06.2006
(51) Int. Cl.: C12N 15/82, C07K 14/415

(54) **AUTOAKTIVIERTES RESISTENZPROTEIN**
AUTOACTIVATED RESISTANCE PROTEIN
PROTEINE DE RESISTANCE AUTOACTIVEE

(30) Priorität: 03.06.2005 DE 102005026045
(43) Veröffentlichungstag der Anmeldung: 27.02.2008
(73) Patentinhaber: KWS Saat AG, 37555 Einbeck (DE)
(72) Erfinder: STAHL, Dietmar Jürgen, 37574 Einbeck (DE)
(74) Vertreter: Pohl, Manfred
(86) Internationale Anmeldenummer: PCT/DE2006/000950
(87) Internationale Veröffentlichungsnummer: WO 2006/128444

(56) Entgegenhaltungen:
- WO-A2-01/29239
- BENDAHMANE ABDELHAFID ET AL: "Constitutive gain-of-function mutants in a nucleotide binding site-leucine rich repeat protein encoded at the Rx locus of potato" PLANT JOURNAL, Bd. 32, Nr. 2, Oktober 2002 (2002-10), Seiten 195-204, XP002411210 ISSN: 0960-7412 in der Anmeldung erwähnt
- DATABASE EMBL [Online] 21. März 2002 (2002-03-21), "Arabidopsis thaliana cDNA clone:RAFL09-20-M08, 5'-end." XP002411224 gefunden im EBI accession no. EM_PRO:AV827853 Database accession no. AV827853
- MEYERS BLAKE C ET AL: "Genome-wide analysis of NBS-LRR-encoding genes in Arabidopsis." PLANT CELL, Bd. 15, Nr. 4, April 2003 (2003-04), Seiten 809-834, XP002411220 ISSN: 1040-4651
- FROST DONNA ET AL: "Tobacco transgenic for the flax rust resistance gene L expresses allele-specific activation of defense responses." MOLECULAR PLANT-MICROBE INTERACTIONS, Bd. 17, Nr. 2, Februar 2004 (2004-02), Seiten 224-232, XP002411226 ISSN: 0894-0282 in der Anmeldung erwähnt

## Beschreibung

Die vorliegende Erfindung betrifft ein autoaktiviertes Resistenzprotein zur Erzeugung einer Resistenz gegenüber Pathogenen bei Pflanzen, ein Verfahren zur Herstellung einer transgenen Pflanze sowie transgene Pflanzen.

Durch Pilze, Viren, Nematoden und Bakterien hervorgerufene Pflanzenkrankheiten bewirken weltweit große Ernteverluste, beeinträchtigen die Qualität der Ernteprodukte und machen einen aufwendigen Einsatz chemischer Pflanzenschutzmittel notwendig, weil die natürlichen Abwehrmaßnahmen der Pflanzen, mit deren Hilfe sie die Mehrzahl potentieller Krankheitserreger abwehren oder deren Ausbreitung verzögern und einschränken können, häufig nicht ausreichen. Zu diesen Abwehrmaßnahmen gehören die hypersensitive Reaktion, der kontrollierte Zelltod des Wirtsgewebes an der Infektionsstelle, die Verstärkung der pflanzlichen Zellwand durch Lignifizierung und Kallosebildung, die Bildung von Phytoalexinen und die Produktion von PR-(pathogenesis-related)-Proteinen. Schlüsselmoleküle für die Aktivierung der induzierten Abwehrmaßnahmen sind die pflanzlichen Resistenzgene (R-Gene). Entsprechend der Flohrschen Gen-für-Gen-Hypothese interagiert das Protein eines R-Gens mit einem korrespondierenden Protein eines mikrobiellen Avirulenzgens (Avr-Gen) und löst dadurch die induzierte Abwehrreaktion aus.

Die Mehrzahl der R-Gene können entsprechend dem Aufbau der von ihnen codierten R-Proteine in 5 Klassen eingeteilt werden (Martin et al, 2003). Die Klasse 1 umfasst nur das Pto-Gen der Tomate, welches für eine Serin/Threonin-Kinase codiert. Die Mehrzahl der pflanzlichen R-Gene gehören jedoch zu der Superfamilie der NBS-LRR-Gene, die für eine "nucleotide binding site" (NBS) und ein "Leucine rich repeat" (LRR) codieren. NBS-LRR-Gene, die an ihrem N-Terminus eine "coiled-coil"-Struktur (CC) wie z.B. einen "Leucine Zipper" aufweisen, werden als CC-NBS-LRR-Gene der Klasse 2 zugeordnet. R-Gene vom CC-NBS-LRR-Typ werden in allen Angiospermen gefunden. Zur Klasse 3 zählen die R-Gene vom TIR-NBS-LRR-Typ, die an dem N-Terminus statt einer CC-Domäne eine Sequenz mit Homologie zu der tierischen TIR-Region ("toll-interleukin-1-receptor") tragen. Die TIR-NBS-LRR-Gene machen zwar 75% der R-Gene in *Arabidopsis thaliana* aus, werden aber nicht in Gräsern und nicht in Zuckerrüben (Tian et al., 2004) gefunden.

Die Cf-Gene der Tomate bilden die 4. Klasse der R-Gene. CF-Proteine haben keine NBS-Domäne, aber eine Transmembran-Domäne (TM) und ein extrazelluläres LRR. Zu der 5. Klasse gehört das Xa21-Protein aus Reis, das aus einer extrazellulären LRR-Domäne, einer Transmembran-Domäne und einer intrazellulären Kinasen-Domäne aufgebaut ist.

Während R-Gene durch die R-Gen-Promotoren nur schwach exprimiert werden, führt eine starke, konstitutive Expression von R-Genen der Klasse 1, 2 und 3 zu einer Aktivierung der pflanzlichen Pathogenabwehr selbst in Abwesenheit des korrespondierenden Aviruienzgenproduktes und damit zur Autoaktivierung des R-Proteins (Tang et al., 1999; Oldroyd and Staskawicz, 1998; Bendahmane et al., 2002).

Generell ist die konstitutive Überexpression von R-Genen in transgenen Pflanzen jedoch mit agronomisch unerwünschten Eigenschaften, wie Mikronekrosen (Tang et al., 1999) oder Kleinwüchsigkeit der Pflanzen (Frost et al., 2004) verbunden.

Eine weitere Möglichkeit der Autoaktivierung von R-Proteinen der Klasse 2 und 3 ist die Mutagenese von speziellen, konservierten Aminosäuremotiven in den vollständigen CC-NBS-LRR bzw. TIR-NBS-LRR Proteinen. Die Mutagenese von Sequenzen in der NBS-bzw. LRR Domäne des Rx-Gens der Kartoffel (Bendahmane et al., 2002) und der NBS-Domäne des L6-Gens von Flachs (Howles et al., 2005) führt zu Mutanten, die in der Abwesenheit des korrespondierenden Avirulenzgens nach transienter Expression den Zelltod auslösen.

Deietionsexperimente mit dem Rx-Gen zeigten, dass Deletionsprodukte bestehend aus der CC-Domäne und Teilen der NBS-Domäne nach ihrer transienten Überexpression ebenfalls noch einen Zelltod auslösen können, der schneller eintritt als bei Verwendung des Vollängen-R-Gens. Diese Deletionsprodukte benötigen neben der CC-Domäne noch den P-Loop, die Kinase-2 und die vollständige Kinase-3a der NBS-Domäne. Dagegen führte eine weitere Verkürzung der NBS-Domäne zu einer im Vergleich mit dem Vollängen-R-Gen langsameren HR-Auslösung (Bendahmane et al. 2002).

Eine Autoaktivierung des L10-Gens aus Flachs, einem R-Gen der Klasse 3, konnte durch die Bildung eines verkürzten TIR-NBS-LRR-Proteins erreicht werden, das aus der TIR-Domäne und 34 Aminosäuren der angrenzenden NBS-Domäne einschließlich dem P-Loop besteht (Frost et al., 2004).

Obwohl mehrere Methoden der Autoaktivierung von R-Genen bekannt sind, wurden bislang noch keine transgenen Pflanzen beschrieben, bei denen die Autoaktivierung von R-Proteinen zu einer erhöhten Pilzresistenz ohne gleichzeitige Beeinträchtigung der agronomische Eigenschaften führt. Versuche, zwei autoaktivierte Vollängenvarianten des L6-Gens jeweils unter der Kontrolle des nativen L6-Resistenzgenpromotors oder eines pilzinduzierten Promotors in Flachs stabil zu transformieren, führten entweder zu normalwüchsigen, pilzanfälligen oder zu kleinwüchsigen, pilzresistenteren Pflanzen (Howles et al., 2005).

Aufgabe der vorliegenden Erfindung ist es daher, die Abwehrleistung einer Pflanze gegenüber Pathogenen so zu verändern, dass die Abwehrreaktionen der Pflanze bei Pathogenbefall zuverlässig aktiviert werden, ohne jedoch die agronomischen Eigenschaften der Pflanze negativ zu beeinflussen.

Erfindungsgemäß erfolgt die Lösung der gestellten Aufgabe durch ein autoaktiviertes Resistenzprotein, das von einer Nukleinsäure codiert wird, die einen begrenzten Teil eines NBS-LRR-Resistenzgens aufweist, der sich vom 5'-Ende des codierenden Bereichs des NBS-LRR-Resistenzgens stromabwärts bis zum Beginn der NBS-Domäne des NBS-LRR-Resistenzgens erstreckt und keinen P-Loop umfasst, wobei das NBS-LRR-Resistenzgen kein TIR-NBS-LRR-Resistenzgen ist.

Der begrenzte Teil des NBS-LRR-Resistenzgens, beginnt bei dem Startcodon für die Translation (ATG-Codon) und reicht bis zur NBS-Domäne, die grundsätzlich durch den P-Loop (Kinase-1a Motiv) gekennzeichnet ist. Für die Funktion dieses Teils des NBS-LRR-Resistenzgens darf der P-Loop nicht umfasst sein. Ebenso wenig sollten andere Abschnitte der NBS- und der LRR-Domäne des NBS-LRR-Resistenzgens nicht mehr vorhanden sein. Allerdings können noch einzelne Nukleotide der NBS-Domäne einschließlich des P-Loops verbleiben, sofern durch diese die Auslösung der HR nicht beeinträchtigt wird.

Unter einem autoaktivierten Resistenzprotein wird ein solches Protein verstanden, das in Abwesenheit eines korrespondierenden Avirulenzgenproduktes zu einer Aktivierung der pflanzlichen Pathogenabwehr führt. Insofern hat die Erfindung den Vorteil, dass zur Ausbildung einer Resistenz gegenüber Pathogenen keine Interaktion zwischen einem Resistenzprotein und einem Avirulenzprotein erforderlich ist, wodurch die Abwehrreaktionen der Pflanze wesentlich direkter und letztendlich zuverlässiger ablaufen können.

Eine Autoaktivierung kann beispielsweise durch eine transiente Überexpression des Resistenzgens erreicht werden. Überexpression bedeutet, dass die Expressionstärke des natürlichen R-Genpromotors soweit überschritten wird, dass die von dem R-Protein regulierte Signaltransduktionskaskade in Abwesenheit des korrespondierenden mikrobiellen Avirulenzgenproduktes aktiviert wird. Dadurch kommt es zur Aktivierung der Pathogenabwehr, die sich in einer teilweisen oder vollständigen Krankheitsresistenz niederschlägt.

Eine Autoaktivierung des Resistenzproteins kann aber auch durch Verkürzung der Vollängen R-Gene BvKWS3_165, BvKWS3_135, Bv13033 und Bv12069 der Zuckerrübe sowie des StR3a Gens der Kartoffel auf den 5'-Bereich, der nur für den NBS-und LRR-Domänen-freien N-Terminus der Proteine einschließlich einer eventuellen CC-Domäne codiert, erreicht werden. NBS-Domäne freier N-Terminus bedeutet in diesem Zusammenhang, dass sich das 5'-Ende des codierenden Bereichs des NBS-LRR-Resistenzgens nur so weit zum 3'-Ende hin erstreckt, dass der P-Loop des NBS-LRR-Resistenzgens nicht in seiner wirksamen Struktur umfasst ist. Im einfachen Falle ist der P-Loop vollständig entfernt. Es können aber auch noch einzelne Nukleötide des P-Loops im verkürzten Resistenzgen verbleiben, sofern durch diese die Auslösung der HR nicht verlangsamt wird. Mit der Verkürzung des NBS-LRR-Resistenzgens auf den N-Terminus werden auch die Kinase-2-, Kinase-3-, GLPC- und MHD-Motive einschließlich der flankierenden Aminosäuren entsprechend der Informationen der Datenbanken Prosite (Bairoch et al., 1996) und Pfam (Sonnhammer et al., 1997) sowie der bei Bendahmane et al. (2002) gegebenen Motivdefinitionen beseitigt.

Die Verwendung der verkürzten R-Gene 165_#176, 135_#147, 13033-#159 und Bv12069 und StR3a-#1-155 führt im Vergleich zu den Vollängen-R-Genen zu einer schnelleren Auslösung von Zelltod im Pflanzengewebe. In Kombination mit einem pathogeninduzierbaren Promotor lässt sich damit eine verbesserte, induzierte Pathogenabwehr herbeiführen. Dies gilt auch für solche R-Proteinen, die durch bekannte Mutationen in der MHD- bzw. VHD-Domäne nicht autoaktiviert werden können, wie die Expression der Gene 135_#147 und BvKWS3_135-D480V zeigen.

Da verkürzte R-Gene im Vergleich zu Vollängen-R-Genen deutlich früher in der Lage sind, Zelltod auszulösen, reicht für die verkürzten R-Gene eine geringere Expression aus, um eine für die Pathogenabwehr kritische Proteinkonzentration zu erreichen, wie für das R-Gen135_#147 gezeigt wird.

Der P-Loop oder das Kinase-1 a Motiv ist gemeinsam mit dem Kinase-2 und Kinase-3 Motiv charakteristisch für ATP oder GTP hydrolysierende Proteine (Traut, 1994) und befindet sich in der NBS-Domäne von NBS-LRR-Genen. Der P-Loop charaktersiert den N-terminalen Bereich der NBS-Domäne (Bendahmane et al., 2002). Die Consensussequenz des P-Loops für die R-Gene Prf, Rx, Rpm1, BvKWS3_135, BvKWS3_133 und BvKWS3_165 ist: (I/V)VG(M/I)GG(L/I/S)GKTT(L/V).

Überraschender Weise hat sich herausgestellt, dass eine besonders gute Autoaktivierung mit Nukleinsäuren möglich ist, die für eine Aminosäuresequenz mit einem Sequenzmotiv DAE codieren. Insbesondere codieren die Nukleinsäuren für das Sequenzmotiv AVLXDAE. Die Sequenzmotive DAE und AVLXDAE finden sich beispielsweise in den SEQ ID NOS: 13 und 15.

Bevorzugte Nukleinsäuresequenzen sind diejenigen aus der folgenden Gruppe:
a) Nukleotidsequenz gemäß SEQ ID NO: 1 oder eine zu der Nukleotidsequenz gemäß SEQ ID NO: 1 komplementäre Nukleotidsequenz oder eine Nukleotidsequenz, die mit der Nukleotidsequenz gemäß SEQ ID NO: 1 oder einer zu der Nukleotidsequenz gemäß SEQ ID NO: 1 komplementären Nukleotidsequenz hybridisiert;
b) Nukleotidsequenz gemäß SEQ ID NO: 2 oder eine zu der Nukleotidsequenz gemäß SEQ ID NO: 2 komplementäre Nukleotidsequenz oder eine Nukleotidsequenz, die mit der Nukleotidsequenz gemäß SEQ ID NO: 2 oder einer zu der Nukleotidsequenz gemäß SEQ ID NO: 2 komplementären Nukleotidsequenz hybridisiert;
c) Nukleotidsequenz gemäß SEQ ID NO: 3 oder eine zu der Nukleotidsequenz gemäß SEQ ID NO: 3 komplementäre Nukleotidsequenz oder eine Nukleotidsequenz, die mit der Nukleotidsequenz gemäß SEQ ID NO: 3 oder einer zu der Nukleotidsequenz gemäß SEQ ID NO: 3 komplementären Nukleotidsequenz hybridisiert;
d) Nukleotidsequenz gemäß SEQ ID NO: 4 oder eine zu der Nukleotidsequenz gemäß SEQ ID NO: 4 komplementäre Nukleotidsequenz oder eine Nukleotidsequenz, die mit der Nukleotidsequenz gemäß SEQ ID NO: 4 oder einer zu der Nukleotidsequenz gemäß SEQ ID NO: 4 komplementären Nukleotidsequenz hybridisiert,
e) Nukleotidsequenz gemäß SEQ ID NO: 16 oder eine zu der Nukleotidsequenz gemäß SEQ ID NO: 16 komplementäre Nukleotidsequenz oder eine Nukleotidsequenz, die mit der Nukleotidsequenz gemäß SEQ ID NO: 16 oder einer zu der Nukleotidsequenz gemäß SEQ ID NO: 16 komplementären Nukleotidsequenz hybridisiert;

Der begrenzte Teil des NBS-LRR-Resistenzgens erstreckt sich bei den bevorzugten Nukleotidsequenzen wie folgt:
SEQ ID NO: 1 von Pos. 124-654
SEQ ID NO: 2 von Pos. 155-598
SEQ ID NO: 3 von Pos. 94-573
SEQ ID NO: 4 von Pos. 194-694

Der hier verwendete Begriff "hybridisieren" bedeutet hybridisieren unter üblichen Bedingungen, wie sie in Sambrook et al. (1989) beschrieben sind, bevorzugt unter stringenten Bedingungen. Stringente Hybridisierungsbedingungen sind beispielsweise: Hybridisieren in 4 x SSC bei 65 °C und anschließendes mehrfaches Waschen in 0,1 x SSC bei 65 °C für insgesamt etwa 1 Stunde. Wenig stringente Hybridisierungsbedingungen sind beispielsweise: Hybridisieren in 4 x SSC bei 37 °C und anschließendes mehrfaches Waschen in 1 x SSC bei Raumtemperatur. "Stringente Hybridisierungsbedingungen" kann auch bedeuten: Hybridisieren bei 68 °C in 0,25 M Natriumphosphat, pH 7,2, 7 % SDS, 1 mM EDTA und 1 % BSA für 16 Stunden und anschließendes zweimaliges Waschen mit 2 x SSC und 0,1 % SDS bei 68 °C.

In bevorzugter Weise stammt das für ein autoaktiviertes Resistenzprotein codierende Resistenzgen aus Zuckerrübe oder Kartoffel.

In weiter bevorzugter Weise codiert die Nukleinsäure für eine Aminosäuresequenz mit einer der Consensussequenzen gemäß SEQ ID NOS: 13 bis 15. Innerhalb der Consensussequenzen können funktionell gleichwertige Aminosäuren gegeneinander ausgetauscht werden, beispielsweise Asp gegen Glu, Leu gegen Ile, Ala oder Val, Arg gegen Lys, Phe gegen Trp.

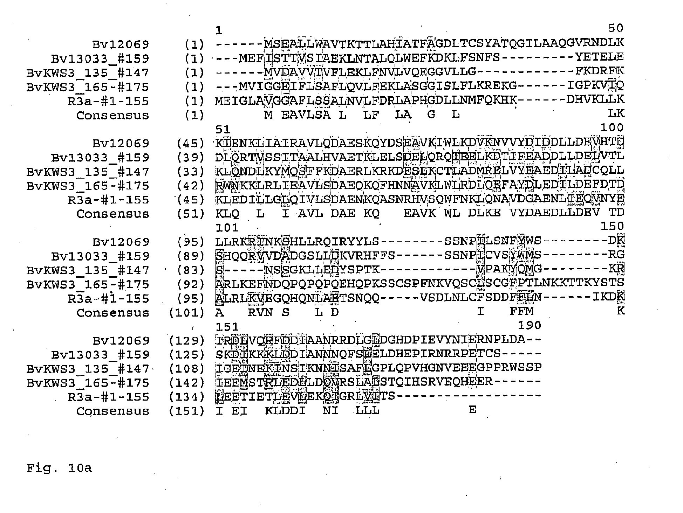

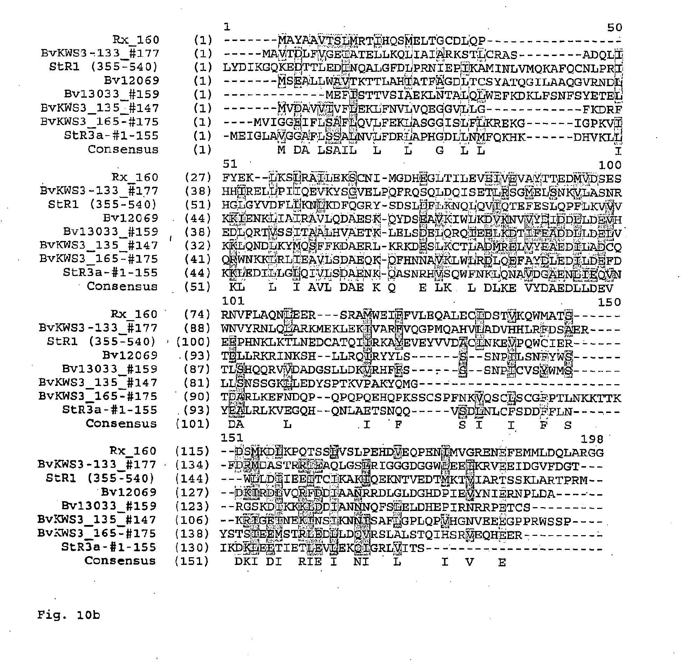

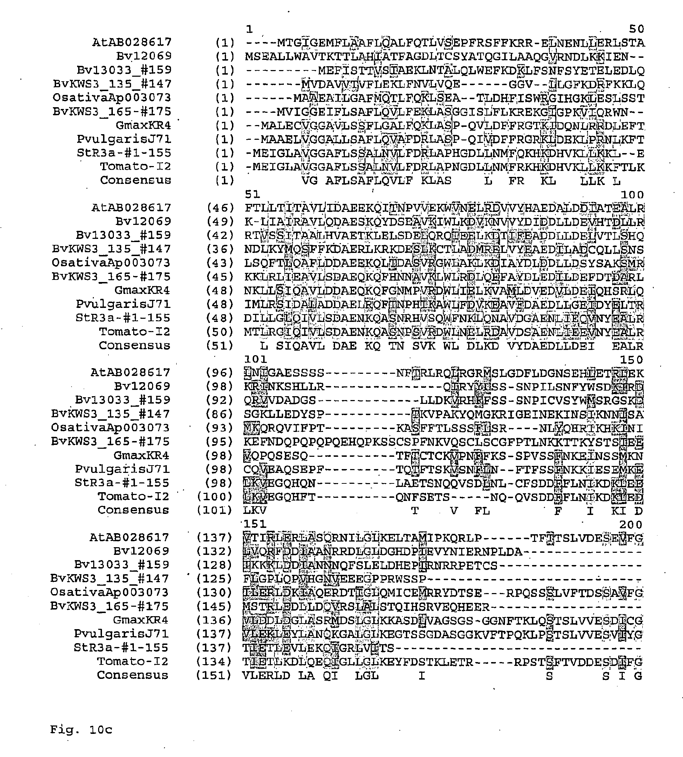

Die beiden Consensussequenzen gemäß SEQ ID NOS: 13 und 14 stellen zwei funktionelle Blöcke dar, die in einem nicht festgelegten Abstand zueinander stehen können. Ein bevorzugter Abstand zwischen beiden Blöcken ergibt sich aus der Consensussequenz gemäß SEQ ID NO: 15 sowie aus der Consensussequenz gemäß Fig. 10.

Die Nukleinsäure lässt sich bevorzugt mit einem pathogeninduzierbaren Promotor kombinieren. Ein pathogeninduzierbarer Promotor wird in Reaktion auf die Infektion des Wirtsgewebes durch einen Krankheitserreger, beispielsweise einen Schadpilz, ein Bakterium, ein Virus oder einen Nematoden aktiviert. Der pathogeninduzierbare Promotor ist während der versuchten oder erfolgreichen Infektion des Pflanzengewebes aktiver als im nicht infizierten Pflanzengewebe.

Pathogeninduzierbare Promotoren sind dem Fachmann grundsätzlich bekannt. Beispiele für pathogeninduzierbare Promotoren sind ein Chitinase Promotor (Samac and Shah 1991), ein Glucanase Promotor (Hennig et al., 1993) und der prp-1 Promotor (Martini et al., 1993).

Durch die pathogeninduzierte Überexpression des R-Gens werden negative Folgen einer konstitutiven Expression wie z.B. Kleinwüchsigkeit oder Minderertrag der Pflanzen vermieden.

Als besonders geeignete Promotoren haben sich synthetische Promotoren erwiesen. Hierbei handelt es sich um durch molekularbiologische Arbeitstechniken erstellte Promotoren, die in dieser Ausführung nicht in der Natur gefunden werden. Ein synthetischer Promotor ist ein minimalistischer Promotor, der neben einem Minimalpromotor nur noch ein oder mehrere ausgewählte, definierte cis-Elemente enthält. Diese cis-Elemente sind Bindungsstellen für DNA-Bindungsproteine wie Transkriptionsfaktoren und werden aus natürlichen Promotoren isoliert, aus bereits isolierten cis-Elementen abgeleitet oder durch zufallsorientierte Rekombinationstechniken technisch erzeugt und durch geeignete Verfahren ausgewählt. Im Vergleich zu einem natürlichen Promotor wird ein synthetischer Promotor aufgrund seines weniger komplexen Aufbau nur durch wenige exogene und endogene Faktoren aktiviert und ist damit spezifischer reguliert.

Der Minimalpromoter oder "Core"-Promoter.ist eine Nukleinsäuresequenz, welche die Bindungsstellen für den basalen Transkriptionsfaktorkomplex enthält und die akkurate Initiation der Transkription durch die RNA-Polymerase II ermöglicht. Charakteristische Sequenzmotive des Minimalpromotors sind die TATA-Box, das Initiatorelement (Inr), das "TFBII recognition element" (BRE) und das "downstream core promoter element" (DPE). Diese Elemente können im Minimalpromotor einzeln oder in Kombination vorkommen. Erhältlich ist der Minimalpromotor oder sind seine Sequenzmotive von einem beliebigen pflanzlichen oder viralen Gen.

Im Rahmen der vorliegenden Erfindung sind neue synthetische Promotoren entwickelt worden, die selbst in Verbindung mit bekannten Resistenzgenen, die nicht zwingend für ein autoaktiviertes Resistenzprotein codieren, zur Herstellung einer pathogenresistenten Pflanze verwendbar sind. Hierbei handelt es sich um Promotoren vom Typ nxS-mxD-Minimalpromotor, nxW2-mxD-Minimalpromotor und nxGst1-mxD-Minimalpromotor, so dass der synthetische Promotor eines oder mehrere der folgenden cis-Elementkombinationen umfasst:
a) eine nxS-mxD-Box
b) eine nxW2-mxD-Box
c) eine nxGst1-mxD-Box
(wobei n und m eine natürliche Zahl von 1...10 bedeuten)

Die S-Box (CAGCCACCAAAGAGGACCCAGAAT) mit der Nukleotidsequenz der SEQ ID NO: 6, die W2-Box (TTATTCAGCCATCAAAAGTTGACCAATAAT) mit Nukleotidsequenz der SEQ ID NO: 7, die D-Box (TACAATTCAAACATTGTTCAAACAAGGAACC) mit der Nukleotidsequenz der SEQ ID NO: 8 und die Gst-Box (TTCTAGCCACCAGATTTGACCAAAC) mit der Nukleotidsequenz der SEQ ID NO: 9 werden bei Rushton et al., 2002 einschließlich der für deren Funktion wichtigen Kemsequenzen beschrieben.

Die Promotoren unterscheiden sich je nach Elementauswahl (nxS-mxD, nxW2-mxD oder nxGst1-mxD) in ihrer Grundaktivität, Pathogeninduzierbarkeit, Aktivierungskinetik und Promotorstärke, wie beispielhaft für Promotoren mit den cis-Elementkombinationen
2xS-2xD mit der Nukleotidsequenz der SEQ ID NO: 10,
2xW2-2xD mit der Nukleotidsequenz der SEQ ID NO: 11 und
2xGst1-2xD mit Nukleotidsequenz der SEQ ID NO: 12
gezeigt wird. Die Eigenschaften eines synthetischen Promotors lassen sich zudem durch die Veränderung der Anzahl der cis-Elemente (n, m = 1...10) entsprechend den Bedürfnissen der Genexpression modifizieren. Der Vergleich des Promotors 2xS-2xD mit den Varianten 2xS-4xD, 4xS-2xD und 4xS-4xD zeigt, dass die durchschnittliche Promotorstärke durch die Verwendung von Tetrameren im Vergleich zu dem aus Dimeren aufgebauten Promotor erhöht wird. Weiterhin steigt die Pathogeninduzierbarkeit vom Dimer-Dimer Promotor (2xS-2xD) über die Tetramer-Dimer und Dimer-Tetramer Promotoren (4xS-2xD, 4xS-2xD) zum Tetramer-Tetramer Promotor (4xS-4xD) zu allen Messzeitpunkten an. Parallel mit der Steigerung der Promotorstärke und der Pathogeninduzierbarkeit kommt es im Fall des beschriebenen Beispiels auch zu einer Erhöhung der Grundaktivität der Tetramere enthaltenden Promotoren. Dieses Beispiel zeigt, dass wichtige Promotoreigenschaften durch die Anzahl der cis-Elemente reguliert werden und für die jeweilige technische Umsetzung optimale Promotoryarianten geschaffen und identifiziert werden können.

Entsprechende Ergebhisse können aber auch mit cis-Elementkombinationen erhalten werden, die Derivate oder Nukleotidsequenz der SEQ ID NO: 10, SEQ ID NO: 11 oder SEQ ID NO: 12 darstellen und über vergleichbare Eigenschaften wie die cis-Elementkombination der SEQ ID NO: 10, SEQ ID NO: 11 oder SEQ ID NO: 12 verfügen.

Die Promotoren 2xS-2xD-Minimalpromoter und 2xW2-2xD-Minimalpromoter wurden beispielhaft jeweils mit den vier Vollängen-R-Genen BvKWS3_133, BvKWS3_123, BvKWS3_135 und BvKWS3_165 kombiniert und in Zuckerrüben transformiert. Eine Pilzresistenzprüfung der transgenen Pflanzen mit dem wichtigsten Schadpilz der Zuckerrübe, *Cercospora beticola,* dem Erreger der Blattfleckenkrankheit, führte mit jedem Konstrukt zu einer verbesserten Pilzresistenz, wobei sich die transgene Pflanze in ihrem Wachstum oder sonstigen agronomischen Eigenschaften nicht von den nichttransgenen Pflanzen unterschieden. Diese Ergebnisse zeigen, dass es unter Verwendung eines pathogeninduzierbaren Promotors grundsätzlich möglich ist, eine Überexpression des Zelltod auslösenden R-Gens und damit eine verbesserte Krankheitsresistenz zu erreichen, ohne dass eine negative Beeinflussung der Pflanzenentwicklung erfolgt. Durch Verwendung von optimierten Promotoren nach Auswahl der günstigsten Anzahl an cis-Elementwiederholungen lässt sich die Krankheitsresistenz noch weiter verbessern.

Die vorliegende Erfindung betrifft ebenfalls transgene Pflanzen, die mit dem neuen Nukleinsäurekonstrukt transformiert wurden, insbesondere Zuckerrübenpflanzen, Teile sowie Samen oder Saatgut von solchen Pflanzen, sowie die Verwendung des neuen Nukleinsäurekonstrukts zur Herstellung einer transgenen Pflanze.

Die Erfindung wird nachfolgend und mit Bezug auf die Figuren und Beispiele näher erläutert.

Die für Zuckerrüben beispielhaft beschriebene Erfindung lässt sich auch problemlos auf andere Nutzpflanzen übertragen, aus denen Resistenzgene isolierbar sind.

### Figuren

Fig. 1 zeigt die Karte des binären Vektors pER-35Sluci, der für die *Agrobacferium tumefaciens* vermittelte transiente Expression von R-Genen, in Zuckerrübenblättern verwendet wurde. Der Vektor trägt ein durch ein Intron unterbrochenes Luciferasegen aus *Photinus pyralis,* welches nicht in *A. tumefaciens* exprimiert werden kann.
Fig. 2 zeigt die Auslösung von Zelltod in Zuckerrübenblättern nach transienter Expression des R-Gens BvKWS3_133 durch *Agrobacterium tumefaciens.* Während die transiente Expression des Konstruktes pER-35Sluci zu einer starken Reportergenaktivität in den Rübenblättern führt, löst die Expression des Konstruktes pER133-35Sluci Zelltod aus, so dass keine Reportergenaktivität gemessen werden kann.
Fig. 3 zeigt den Vektor pCaMV-2 der für die transiente, biolistische Transformation der Zuckerrübenblätter verwendet wurde. Die Vollängen- und verkürzten R-Gene wurden wie beschrieben unter die Kontrolle des doppelten 35S Promotors dieses Vektors gestellt.

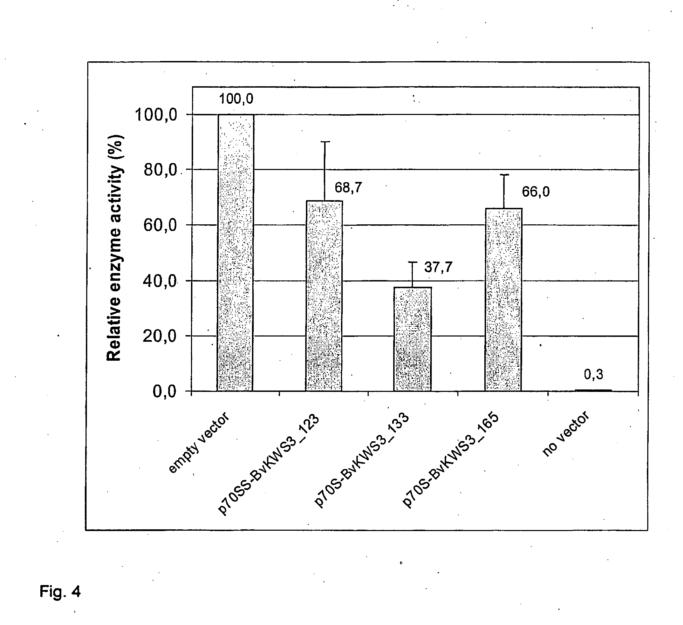

Fig. 4 zeigt die Auslösung von Zelltod in Zuckerrübenblättern nach transienter Expression der R-Gene BvKWS3_123, BvKWS3_133 und BvKWS3_165 durch biolistische Transformation. Die Gene BvKWS3_123, BvKWS3_133 und BvKWS3_165 stehen unter der Kontrolle des doppelten 35S-Promotors (d35S) und wurden mit dem Reportergenkonstrukt p70S-luc cotransformiert. Die Reportergenaktivität wurde 20 h nach Transformation gemessen. Durch Auslösung einer hypersensitiven Reaktion ist die Reportergenaktivität im Vergleich zu der Kontrolle (Leervektor pCaMV-2 und p70S-luc) reduziert. Dargestellt ist der Mittelwert von 3 unabhängigen Versuchswiederholungen mit jeweils 9 Einzelexperimenten pro Konstrukt. Der Fehlerbalken gibt den Standardfehler an.
Fig. 5 zeigt eine verstärkte Zelltodauslösung durch die Expression des 5'-terminalen Bereiches des R-Gens BvKWS3_165 im Vergleich zu der Expression des Vollängen-R-Gens BvKWS3_165. Der N-terminale Bereich und das Vollängen-R-Gen wurden unter der Kontrolle des d35S Promotors (p70S-165_#175 und p70S-BvKWS3_165) mit dem Konstrukt p70S-luc durch bioloistische Transformation in Zuckerrübenblättercotransformiert. Dargestellt ist der Mittelwert von 3 unabhängigen Versuchswiederholungen mit jeweils 9-12 Einzelexperimenten pro Konstrukt.
Fig. 6 zeigt eine Zelltodauslösung durch die Expression des Vollängen R-Gens BvKWS3_135 und eine im Vergleichs dazu verstärkte Zelltodauslösung durch den 5'-terminalen Bereich 135_#147 des R-Gens BvKWS3_135. Das Vollängen R-Gen und der N-terminale Bereich 135_#147 wurden unter der Kontrolle des d35S Promotors (p70S-BvKWS3_135 und p70S-135_#147) mit dem Konstrukt p70S-luc durch bioloistische Transformation in Zuckerrübenblätter cotransformiert. Dargestellt ist der Mittelwert von 2 unabhängigen Versuchswiederholungen mit jeweils 9-12 Einzelexperimenten pro Konstrukt.
Fig. 7 zeigt eine verstärkte Zelltodauslösung durch die Expression des 5'-terminalen Bereiches 13033_#159 des R-Gens Bv13033 im Vergleich zu der Expression des Vollängen-R-Gens Bv13033. Das Vollängen-R-Gen und der N-terminale Bereich 13033_#159 wurden unter der Kontrolle des d35S Promotors (p70S-13033 und p70S-13033_#159) mit dem Konstrukt p70S-luc durch bioloistische Transformation in Zuckerrübenblätter cotransformiert. Dargestellt ist der Mittelwert von 2 unabhängigen Versuchswiederholungen mit jeweils 9-12 Einzelexperimenten pro Konstrukt.
Fig. 8 zeigt die Auslösung von Zelltod durch die Expression des R-Gen Bv12069.
Fig. 9 zeigt die Autoaktiverung des Proteins BvKWS3_135 durch Verkürzung auf den 5'-Bereich des cDNA Klons 135_#147 im Vergleich zur Mutation des VHD Motivs der NBS Domäne.
Fig. 10a)-c) zeigen Vergleiche der Aminosäuresequenzen der verkürzten, autoaktiven Proteine Bv12069, Bv13033_#159. BvKWS135_#147, BvKWS3_165_#175 und StR3a-#1-155 untereinander sowie mit Vergleichssequenzen nichtautoaktiver verkürzter Resistenzproteine aus Kartoffel (RX-160) und StR1 (355-540) sowie vollständiger R-Proteine des NBS-LRR Typs aus Arabidopsis thaliana (AtAB028617), Bohne (PvulgarisJ71), Reis (OsativaAP003073), Sojabohne (GmaxKR4) und der Tomate (Tomato-I2). Consensussequenzen sind hervorgehoben.
Fig. 11 zeigt, dass die Deletion des Aminosäuren 147-175 die Autoaktivierbarkeit des Proteins 165_#175 deutlich reduziert.
Fig. 12 zeigt die Aktivierung des synthetischen Promotors 2xS-2xD in transgenen Zuckerrüben nach *Cercospora beticola*-Befall.
Fig. 13 zeigt die Aktivierung des synthetischen Promotors 2xW2-2xD in transgene Zuckerrüben nach *Cercospora* beticola-Befall.
Fig. 14 zeigt den Vergleich der Reportergenaktivität der Promotoren 2xS-2xD, 4xS-2xD, 2xS-4.xD und 4xS-4xD in transgenen Zuckerrüben nach Cercospora beticola-Befall.
Fig. 15 und 16 zeigt Kombinationen der Vollängen R-Gene 123, 133, 135, 165 mit dem synthetischen Promotor 2xS-2xD.
Fig. 17 und 18 zeigt Kombinationen der Vollängen R-Gene 123, 133, 135, 165 mit dem synthetischen Promotor 2xW₂-2xD.
Fig. 19 zeigt die Resistenzsteigerung der transgenen Zuckerrübenlinie PR68-6 gegenüber dem Schadpilz *Cercospora beticola* im Vergleich zu den nichttransgenen Kontrolle 3DC4156.
Fig. 20 zeigt die Resistenzsteigerung der transgenen Zuckerrübenlinie PR70-32 gegenüber Cercospora beticola im Vergleich zu den nichttransgenen Kontrolle 3DC4156.
Fig. 21 und 22 zeigt die Kombination der N-terminalen Bereiche der R-Gene, 165_#176 und 12069 mit den synthetischen Promotoren 2xS-2xD und 2xW₂-2xD.

### Beispiele

### Nachweis der Auslösung einer schnellen Resistenzreaktion in Zuckerrübenblättern durch Überexpression des Gens BvKWS3 133

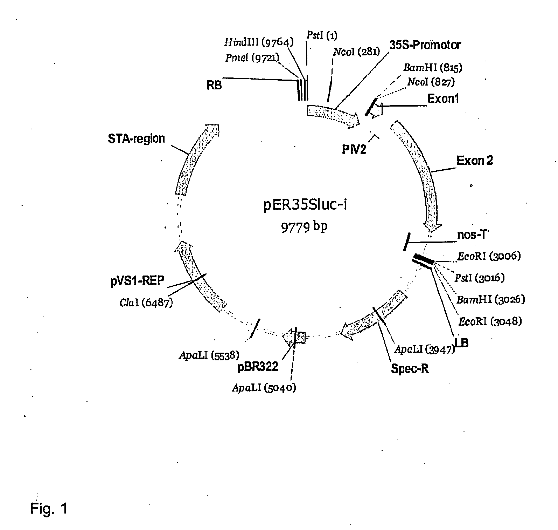

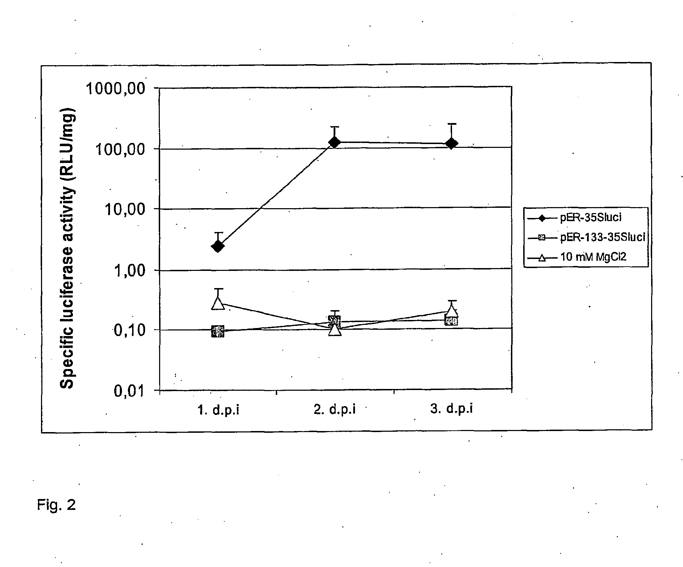

Die transiente Überexpression des Vollängen cDNA Klons des Gens BvKWS3_133 in Zuckerrübenblättern durch *Agrobacterium tumefaciens* löst einen schnellen Zelltod ohne sichtbare Nekrosenbildung aus. Der cDNA-Klon BvKWS3_133 wurde mit dem d35S Promotor kombiniert und in den binären Vektor pER-35Sluci (Fig. 1) inseriert. Der entstehende Vektor trägt die Bezeichnung pER133-35Sluci. Die Vektoren pER-35Sluci und pER133-35Sluci wurden in den *Agrobacterium* Stamm C58C1 transformiert (An 1987). Positive Agrobacterien wurden für die transiente Expression in 50 ml LB-Medium mit 100 mg/ml Spectinomycin und 20 µM Acetosyringon 4-5 h angezogen. Anschließend wurden die Bakterien abzentrifugiert und das Sediment in einer Lösung von 10 mM MgCl₂, 10 mM MES, 100 µM Acetosyringon aufgenommen und eine Bakteriendichte von OD₆₀₀ = 0,1 eingestellt. Die Bakteriensuspension wurde 2-3 h ruhen gelassen und dann mit Hilfe einer 2,5 ml Spritze über die Blattunterseite von 10 Wochen alten Zuckerrüben in die Blätter injiziert. Nach Inkubation bei 25°C in einem Kulturschrank wurde am 1, 2 und 3 Tag nach Inokulation die *Photinus pyralis* Luciferasereportergenaktivität in den transformierten Blättern gemessen. Dazu wurde die Luciferaseaktivität mit dem Luciferase Assay System (Promega, Mannheim, Deutschland) in einem Sirius Luminometer (Berthold Detection System GmbH, Pforzheim, Deutschland) entsprechend den Herstellerangaben bestimmt. Für die Gewinnung eines für die Messung geeigneten Enyzmextraktes wurden pro Messpunkt jeweils zwei Blattrondelle ausgestanzt. Pro Konstrukt wurden 8 Meßpunkte pro Messtag entnommen. Die Blattproben werden unter Zugabe von Seesand mit dem 10-fachen Volumen (v/w) an Passive Lysis Buffer (PLB) in einem Mörser homogenisiert. Der flüssige Überstand wurde in ein 1.5-ml-Eppendorfgefäß überführt und 5 min bei 4 °C und 20 000 g abzentrifugiert. Der klare Überstand wurde abgenommen und jeweils 10 µl Rohextrakt für die Photinus-Luciferaseaktivitätsmessung eingesetzt. Zuckerrübenblätter, die mit dem Kontrollkonstrukt pER-35Sluci transformiert worden waren, zeigten am 1. Tag eine geringe und am 2. und 3. Tag eine Luciferaseaktivität von 124.000 bzw. 116.000 RLU/mg Blattgewebe. Rübenblätter, die mit dem Konstrukt pER133-35Sluci transformiert wurden, zeigten zu allen 3 Messzeitpunkten keine Aktivität, die größer als die der MgCl₂ inokulierten Blättern war (Fig. 2). Damit löst die transiente Expression des cDNA Klons BvKWS3_133 einen sehr schnellen Zelltod in den inokulierten Rübenblättern aus.

### Die konstitutitve Expression der R-Gene BvKWS3 123, BvKWS3 133 und BvKWS3 165 löst einen Zelltod in Zuckerrübenblättern aus

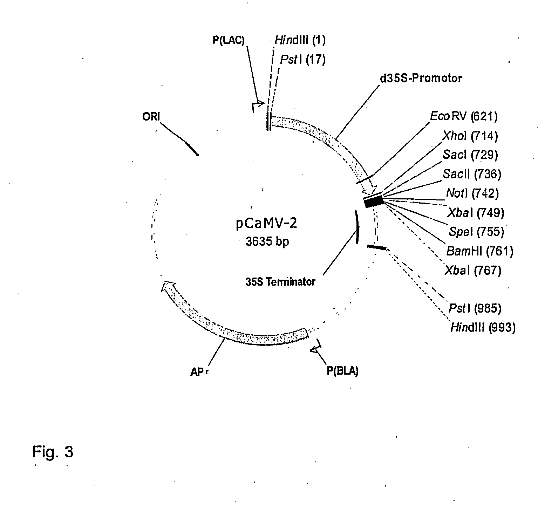

Das R-Gen BvKWS3_133 sowie das R-Gen BvKWS3_165 mit der Nukleotidsequenz gemäß SEQ ID NO: 5 und das R-Gen BvKWS3_123 wurden mit dem doppelten 35S Promotor des Vektors pCaMV-2 (Fig. 3) kombiniert. Die entstandenen Vektoren tragen die Bezeichnung p70S-BvKWS3_133, p70S-BvKWS3_165 und p70S-BvKWS3_123. Um die Funktionalität der R-Gene zu prüfen, wurden die Konstrukte p70S-BvKWS3_133, p70S-BvKWS3_165 und p70S-BvKWS3_123 mit dem Reportergenvektor p70S-luc in Zuckerrübenblättern transient durch biolistische Transformation nach Schmidt et al., 2004 exprimiert. Als Positivkontrolle wurde der Leervektor pCaMV-2 in Kombination mit dem Reportergenvektor p70S-luc verwendet. Auf die Verwendung eines Normalisierungsvektors wurde abweichend von Schmidt et al. (2004) verzichtet. Die Luciferaseaktivität wurde mit dem Luciferase Assay System (Promega,'Mannheim, Deutschland) 20 h nach der Transformation bestimmt. Die Transformationsexperimente wurden dreimal wiederholt, wobei jedes Experiment 9 Versuchswiederholungen pro Konstrukt umfasste. Die Bildung des Mittelwertes aus den 3 Experimenten zeigte, dass im Vergleich zu der gleich 100% gesetzten Luciferaseaktivität der Positivkontrolle (empty vector), die Reportergenaktivität bei p70S-BvKWS3_133 nur 37,7% , bei p70S-BvKWS3_165 nur 66% und bei p70S-BvKWS3_123 nur 68,7% betrug (Fig. 4). Die starke Expression der R-Gene BvKWS3_133, BvKWS3_165 und BvKWS3_123 durch den d35S Promotor löst somit Zelltod bzw. eine Hypersensitive Reaktion in einem Teil der transformierten Zellen aus, die eine Coexpression des mittransformierten Reportergenvektors verhindert. Damit wurde gezeigt, dass die starke Expression der drei R-Gene zu einem Zelltod bzw. einer HR in der Abwesenheit eines korrespondierenden Aviruelnzgenproduktes führt.

### Der 5'-. Bereich des Gens BvKWS3 165 löst einen schnelleren Zelltod aus als der vollständige cDNA Klon BvKWS3 165

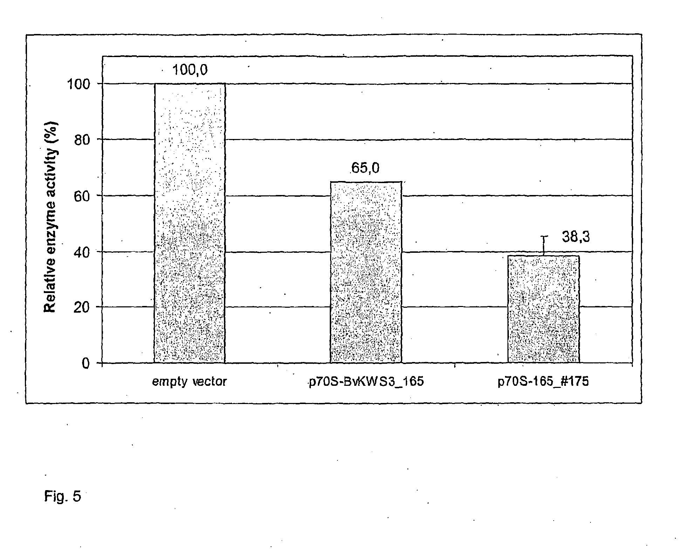

Ausgehend vom Vollängen cDNA Klon BvKWS3_165 mit der Nukleotidsequenz gemäß SEQ ID NO: 5 in dem Konstrukt p70S-BvKWS3_165 wurde der 5'-Bereich des Gens mit Hilfe der Pfu-Polymerase (Stratagene) unter Verwendung der Primer S316 (CTGGAGAATTCGAGCTCCACCGCGG) und S318 (CTGGATCCTCACCTCCGTTCTTCATGTTGCTCTACC) amplifiziert und gleichzeitig ein Stoppcodon in den codierenden Bereich eingeführt. Der amplifizierte Bereich entspricht der Nukleotidsequenz gemäß SEQ ID NO: 1 und codiert für die Aminosäuresequenz 1-175 von BvKWS3_165 (Fig. 10). Die Aminosäuresequenz umfasst nur den N-terminalen Bereich von BvKWS3_165 und enthält keine NBS und keine LRR Domäne (Fig. 10). Das PCR Produkt wurde mit den Restriktionsenzymen Sacll und BamHI geschnitten und in den Vektor pCaMV-2 kloniert. Der resultierende Vektor trägt die Bezeichnung p70S-165_#175. Die Eignung der Konstrukte p70S-BvKWS3_165 und p70S-165_#175 einen Zelltod in Zuckerrübenblätter auszulösen, wurde durch transiente, bioloistische Transformationen quantitativ getestet. Dazu wurde jeder Vektor mit dem Reportergenvektor p70S-luc cotransformiert. Als Positivkontrolle wurde der Leervektor pCaMV-2 in Kombination mit dem Reportergenvektor p70S-luc verwendet.
Im Vergleich zur Transformation des Leervektors (pCaMV-2) führt die Transformation von p70S-BvKWS3_165 zu 65% messbarer Reportergenaktivität und die Transformation von p70S-165_#175 nur zu 38% messbarer Reportergenaktivität (Fig. 5). Dieses Ergebnis zeigt, dass die exklusive Expression des 175 Aminosäuren großen N-Terminus von 165_#175 zu einer intensiveren Auslösung von Zelltod in dem transformierten Zuckerrübenblättern führt als die Verwendung des 1066 Aminosäuren großen Vollängenproteins BvKWS3_165. Durch Expression von 165_#175 sterben mehr der transformierten Blattzellen ab als im Fall der Expression von BvKWS3_165. Ursache für diesen Unterschied ist eine neue, intensivere Form der Autoaktivierung des R-Proteins durch eine Verkürzung auf den N-Terminus.

### Der 5'- Bereich des Gens BvKWS3 135 löst einen schnelleren Zelltod aus als der vollständige cDNA Klon BvKWS3 135

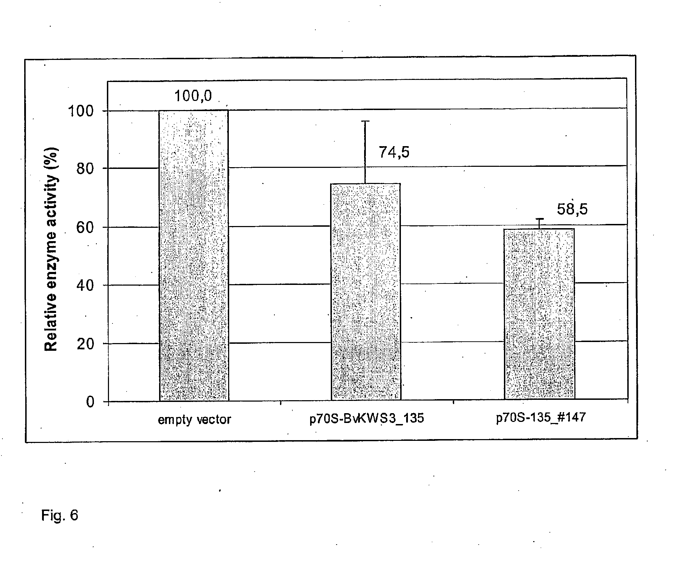

Ausgehend vom Vollängen cDNA Klon BvKWS3_135 in dem Konstrukt p70S-BvKWS3_135 wurde der 5'-Bereich des Gens mit Hilfe der Pfu-Polymerase (Stratagene) unter Verwendung der Primer S316 (CTCGAGAATTCGAGCTCCACCGCGG) und S330 (CTGGATCCTCAGGGAGAACTCCATCTGGGTGGTCC) amplifiziert und gleichzeitig ein Stoppcodon in den codierenden Bereich eingeführt. Der amplifizierte Bereich entspricht der Nukleotidsequenz gemäß SEQ ID NO: 2 und codiert für die Aminosäuresequenz 1-147 von BvKWS3_135 (Fig. 10). Die Aminosäuresequenz umfasst nur den N-terminalen Bereich von BvKWS3_135 und enthält keine NBS und keine LRR Domäne bzw. Motive aus diesen Domänen. Das PCR Produkt wurde mit den Restriktionsenzymen Sacll und BamHI geschnitten und in den Vektor pCaMV-2 kloniert. Der resultierende Vektor trägt die Bezeichnung p70S-135_#147. Die Fähigkeit der Konstrukte p70S-BvKWS3_135 und p70S-135_#147 einen Zelltod in Zuckerrübenblätter auszulösen, wurde durch transiente, bioloistische Transformationen quantitativ getestet. Dazu wurde jeder Vektor mit dem Reportergenvektor p70S-luc cotransformiert. Als Positivkontrolle wurde der Leervektor pCaMV-2 in Kombination mit dem Reportergenvektor p70S-luc verwendet. Im Vergleich zur Transformation des Leervektors (pCaMV-2) führt die Transformation von p70S-BvKWS3_135 zu 74,5% messbarer Reportergenaktivität und die Transformation von p70S-135_#147 nur zu 58,5% messbarer Reportergenaktivität (Fig. 6). Die Ergebnisse zeigen, dass die Expression des Vollängenklons BvKWS3_135 zur Auslösung von Zelltod in dem transformierten Gewebe führt. Allerdings bewirkt die exklusive Expression des 147 Aminosäuren großen N-Terminus von 135_#147 einen intensiveren Zelltod in den transformierten Zuckerrübenblättern als die Verwendung des 844 Aminosäuren großen Proteins BvKWS3_135. Durch Expression von 135_#147 sterben mehr transformierte Blattzellen ab als im Fall der Expression von BvKWS3_135. Ursache für diesen Unterschied ist eine neue, intensivere Form der Autoaktivierung des R-Proteins durch eine Verkürzung auf den N-Terminus.

### Der 5'- Bereich des Gens Bv13033 löst einen schnelleren Zelltod aus als der vollständige cDNA Klon Bv13033

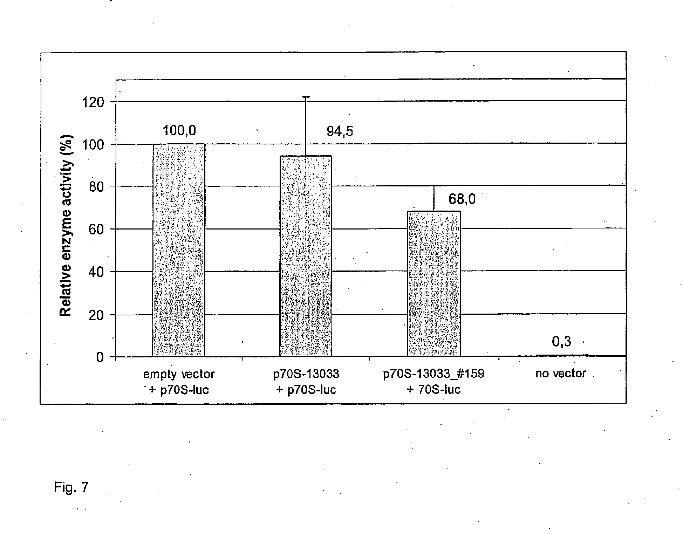

Ausgehend vom Vollängen cDNA Klon Bv13033 in dem Konstrukt p70S-13033 wurde der 5'-Bereich des Gens mit Hilfe der Pfu-Polymerase (Stratagene) unter Verwendung der Primer S316 (CTCGAGAATTCGAGCTCCACCGCGG) und S333 (CTGGATCCTCAAGAACAAGTCTCAGGCCTTCTGTT) amplifiziert und gleichzeitig ein Stoppcodon in den codierenden Bereich eingeführt. Der amplifizierte Bereich entspricht der Nukleotidsequenz gemäß SEQ ID NO: 3 und codiert für die Aminosäuresequenz 1-159 von Bv13033 (Fig. 10). Die Aminosäuresequenz umfasst nur den N-terminalen Bereich von Bv13033 und enthält keine NBS und keine-LRR Domäne oder Motive aus diesen Domänen. Das PCR Produkt wurde mit den Restriktionsenzymen Sacll und BamHI geschnitten und in den Vektor pCaMV-2 kloniert. Der resultierende Vektor trägt die Bezeichnung p70S-13033_#159. Die Eignung der Konstrukte p70S-13033 und p70S-13033_#159 einen Zelltod in Zuckerrübenblätter auszulösen, wurde durch transiente, biolistische Transformationen quantitativ getestet. Dazu wurde jeder Vektor mit dem Reportergen-vektor p70S-luc cotransformiert. Als Positivkontrolle wurde der Leervektor pCaMV-2 verwendet. Im Vergleich zur Transformation des Leervektors (pCaMV-2) führt die Transformation von p70S-13033 zu 95% messbarer Reportergenaktivität und die Transformation von p70S-165_#175 nur zu 68% messbarer Reportergenaktivität (Fig. 7). Diese Ergebnisse zeigen, dass die Expression des Vollängenklons Bv13033 nur zur Auslösung eines schwachen Zelltods in dem transformierten Gewebe führt. Die exklusive Expression des 159 Aminosäuren großen N-Terminus von 13033_#159 bewirkt hingegen einen intensiven Zelltod in den transformierten Zuckerrübenblättern. Ursache für diesen Unterschied ist eine neue, intensivere Form der Autoaktivierung des R-Proteins durch eine Verkürzung auf den N-Terminus.

### Auslösung von Zelltod in Zuckerrübenblättern durch den 5'- Bereich des Gens Bv12069

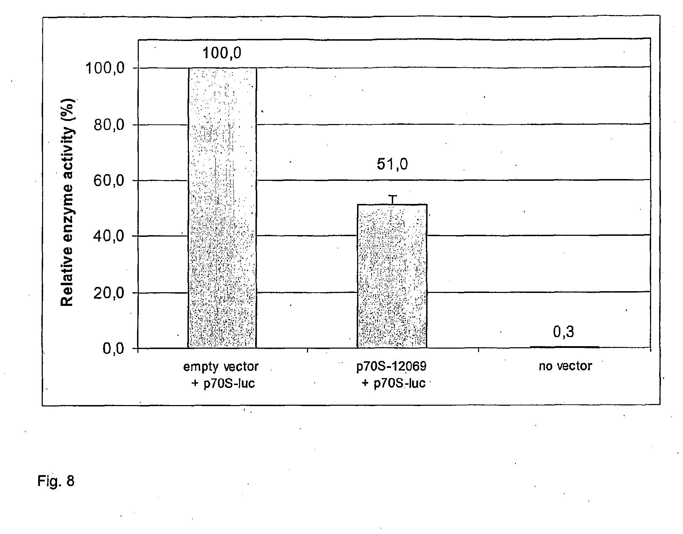

Das R-Gen Bv12069 mit der Nukleotidsequenz gemäß SEQ ID NO: 4 codiert für den 166 Aminosäuren großen N-Terminus eines R-Proteins. Das Protein Bv12069 enthält keine NBS und keine LRR Domäne, zeigt aber eine deutliche Homologie zu den 175, 147 und 159 Aminosäuren großen N-Termini der autoaktivierten R-Proteine 165_#175, 135_#147 13033_#159 (Fig. 10). Der cDNA Klon wurde mit dem doppelten 35S Promotor des Vektors pCaMV-2 (Fig. 3) zu dem Vektor p70S-12069 kombiniert. Um die Funktionalität des Gens Bv12069 zu prüfen, wurde das Konstrukt p70S-12069 in Kombination mit dem Reportergenvektor p70S-luc in Zuckerrübenblättern transient durch biolistische Transformation exprimiert. Die Reportergenaktivität in den mit p70S-12069 und p70S-luc transformierten Blättern betrug in drei unabhängigen Versuchen nur 51 % der Aktivität, die in der Positivkontrolle (Leervektor pCaMV-2 und p70S-luc) gemessen werden konnte (Fig. 8). Die Expression des 166 Aminosäuren großen Proteins Bv12069 löst somit einen Zelltod in Zuckerrübenzellen aus.

### Das Verkürzung des Gens BvKWS3 135 führt zu einem autoaktivierten R-Protein nicht jedoch die Mutagenese der MHD Domäne

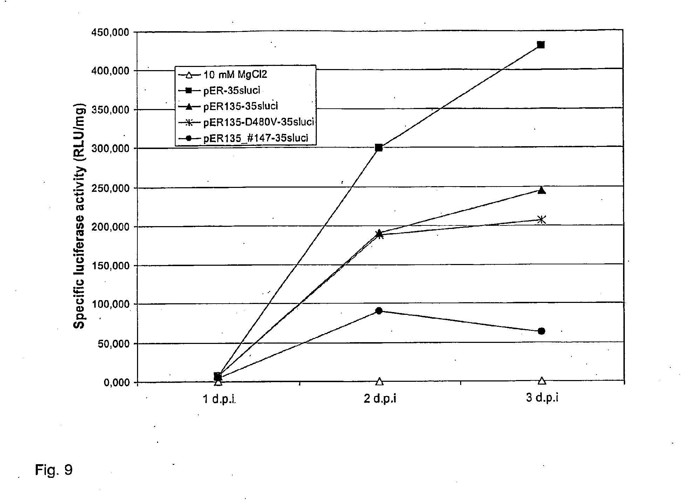

Der erfindungsgemäße Mechanismus der Autoaktivierung durch Verkürzung eines R-Proteins vom NBS-LRR Typ auf den NBS- und LRR freien N-Terminus wurde mit der Methode der Autoaktivierung durch Mutagenese des MHD Motives verglichen: Die Mutagenese des MHD Motives des Rx-Gens der Kartoffel und des L6 Gens aus Flachs führte zu einer Autoaktivierung der genannten Gene (Bendahmane et al., 2002; Howles et al., 2005). Der cDNA Klon BvKWS3_135 codiert für das dem MHD Motiv entsprechende VHD Motiv, ein Motiv das neben dem MHD Motiv ebenfalls häufig in R-Genen gefunden wird (Howles et al., 2005). Die entsprechende Mutation wurde wie von Bendahmane et al. (2002) beschrieben in den Vollängenklon BvKWS3_135 eingeführt. Dazu wurde die Aminosäure Aspartat im VHD Motiv des Gens BvKWS3_135 durch die Aminosäure Valin ausgetauscht. Das entstehende Gen trägt die Bezeichnung BvKWS3_135_D480V. Die Wirksamkeit der Gene 135_#147, BvKWS3_135_D480V und des nichtmodifizierten Gens BvKWS3_135 wurde durch *Agrobacterium tumefaciens* vermittelte transiente Überexpression in Zuckerrübenblättern überprüft. Dazu wurde der cDNA Klon BvKWS3_135 mit dem d35S Promotor kombiniert und in den binären Vektor pER-. 35Sluci inseriert. Der entstehende Vektor trägt die Bezeichnung pER135-35Sluci. Entsprechend wurde mit dem verkürzten cDNA Klon 135_#147 mit der Nukleotidsequenz gemäß SEQ ID NO:2 sowie mit dem mutagenisierten cDNA Klon BvKWS3_135_D480V Verfahren. Die entstehenden Vektoren tragen die Bezeichnung pER135_#147-35Sluci und pER135_D480V-35Sluci. Die Vektoren wurden wie beschrieben in den Agrobacterium Stamm C58C1 transformiert und gemeinsam mit der Positivkontrolle pER-35Sluci in Zuckerrübenblätter injiziert. Die *Photinus pyralis* LuciferaseReportergenaktivität wurde 1, 2 und 3 Tage nach der Inokulation in den transformierten Blättern gemessen. Zuckerrübenblätter, die mit dem Kontrollkonstrukt pER-35Sluci transformiert worden waren, zeigten am 1. Tag eine geringe und am 2. und 3. Tag eine Luciferaseaktivität von 299.000 und 433.000 RLU/mg Blattgewebe. Rübenblätter, die mit dem Konstrukt pER135-35Sluci transformiert wurden, zeigten am 2. und 3. Tag eine Luciferaseaktivität von 190.000 und 245.000 RLU/mg Blattgewebe und damit im Vergleich zu der Positivkontrolle pER-35Sluci einen messbaren Zelltod. Die Reportergenaktivität des Konstruktes pER_135_D480V-35Sluci betrug am 2. und 3. Tag 188.000 und 206.000 RLU/mg (Fig. 9). Damit führte die Einführung der MHD Mutation in das Gen BvKWS3_135 zu keiner bzw. einer kaum messbaren Autoaktivierung. Das entsprechend diesem Verfahren verkürzte R-Gen 135_#147 hingegen zeigte am 2. und 3. Tag eine Reportergenaktivität von 90.000 und 63.000 RLU/mg (Fig. 9) und damit eine deutlich stärkere Zelltodauslösung und Autoaktivierung als die Konstrukte pER135-35Sluci und pER_135_D480V-35Sluci.

### Identifizierung gemeinsamer Aminosäuremotive in den N-Termini der R-Proteine von BvKWS3 165, BvKWS3 135, Bv13033 und Bv12069 und StR3a

Ein Homologievergleich zwischen den 175, 147, 159 und 166 Aminosäuren großen N-Termini der R-Proteine BvKWS3_165, BvKWS3_135, Bv13033 und Bv12069 und des 155 Aminosäure großen N-Terminus des R3a Gens der Kartoffel (Huang et al., 2005) wurde durchgeführt, um gemeinsame Sequenzmotive zu identifizieren. Der Vergleich führte zu der Identifizierung mehrerer Konsensussequenzen in den N-Termini der autoaktivierten R-Proteine. Die gemeinsamen Sequenzmotive sind als Konsensussequenz in der Fig. 10a) hervorgehoben.

Eine Konsensussequenz entspricht der Aminosäuresequenz gemäß SEQ ID NO: 13: AVLXDAEXKQXX XXXLXXWLXD LKDXVYDXDD ILDE. Eine andere Konsensussequenz entspricht der Aminosäuresequenz gemäß SEQ ID NO: 14: IXEIXXKLDD L

Der Büchstabe X steht hierbei für eine beliebige Aminosäure.

Beide Konsensussequenzen sind in der beschriebenen Form nur in solchen N-Termini von CC-NBS-LRR R-Proteinen enthalten, deren Expression zu einer Autoaktivierung führt. So ist die 160 Aminosäure große CC-Domäne des RX-Gens nicht in der Lage, Zelltod bzw. eine hypersensitive Reaktion auszulösen (Bendahmane et al., 2002). Die transiente Expression des 177 Aminosäure großen N-Terminus des R-Gens BvKWS3_133_e08 der Zuckerrübe und des 540 Aminosäure großen N-Terminus des R1 Gens der Kartoffel (Ballvora et al., 2002) löste im Vergleich zum vollständigen R-Gen BvKWS3_133_e08 keinen verstärkten oder im Falle des R1 Gens keinen Zelltod aus (Daten nicht gezeigt). Der Aminosäurevergleich der N-Termini der autoaktivierten Proteine BvKWS3_165_#176. BvKWS3_135_#147. Bv13033_#159, Bv12069 und StR3a-#1-155 mit den Aminosäuresequenzen der CC-Domänen der Rx-, StR1- und BvKWS3__133_#177-Proteine zeigt die Abwesenheit der oben beschriebenen Konsensussequenzen in den nicht autoaktivierten N-Termini (Fig. 10b). Insbesondere ist das Sequenzmotiv DAE ein wichtiges Hilfsmittel zur Identifizierung von R-Proteinen, deren N-Terminus autoaktiv ist. Mit Hilfe des Sequenzmotivs DAE in der Konsensussequenz lassen sich in zahlreichen Pflanzenspezies geeignete R-Gene für eine Autoaktivierung finden, wie Fig. 10c für Beispiele aus Arabidopsis thaliana (AtAB028617), Bohne (PvulgarisJ71), Reis (osativaAp003073), Sojabohne (GmaxKR4) und Tomate (Tomato-12) zeigt.

### Die Aminosäureseauenz von 147-175 ist wichtig für die Autoaktivierung des R-Proteins 165 #175

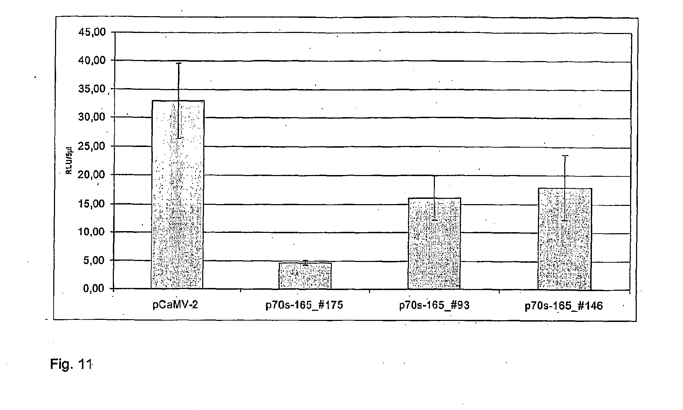

Um die Aminosäureabschnitte in dem Protein 165_#175 zu identifizieren, die für die Autoaktivierung des N-Terminus von NBS-LRR Proteinen wichtig sind, wurde der codierende Bereich des cDNA Klons 165_#175 verkürzt. Die cDNA Klone 165_#93 und 165_#146 codieren für die Aminosäuren 1-93 bzw. 1-146 des Proteins 165_#175. Der transiente biolistische Test der Konstrukte p70S_165_#93, p70S_165_#146 und p70S_165_#175 zeigte, dass nur das Protein 165_#175, nicht aber 165_#93 und 165_#146 eine starken Zelltod auslösen (Fig. 11). Damit ist der Sequenzbereich von 146-175 essentiell für die Autoaktivierung von NBS-LRR Proteinen. In diesem Bereich liegt ein zwischen allen untersuchten Proteinen konserviertes Sequenzmotiv (Fig. 10a)).

### Schnelle Aktivierung der synthetischen pathogeninduzierbaren Promotoren 2xS-2xD und 2xW2-2xD durch Pilzbefall

Für die pathogeninduzierte Überexpression von vollständigen oder partiellen Resistenzgenen eigenen sich besonders synthetische Promotoren vomTyp nxS-mxD, nxW2-mxD und nxGst1-mxD, wobei n=1,2,3,4,5,6,7,8,9,10 und m=1,2,3,4,5,6,7,8,9,10 ist. Beispielhaft wurden Promotoren vom Typ 2xS-2xD gemäß SEQ ID No. 10, 2xW2-2xD gemäß SEQ ID No. 11 sowie und 2xGst1~2xD gemäß SEQ ID No. 12 mit dem Luciferasegen aus *Photinus pyralis* kombiniert, in Zuckerrüben transformiert und in Reaktion auf Pilzbefall analysiert.
Für die Pflanzentransformation fanden die binären Vektoren 2xS-2xD-luc-kan, 2xW2-2xD-luc-kan, und 2xGst1-2xD-luc-kan Verwendung. Die binären Vektoren wurden in den *Agrobacterium tumefaciens* Stamm C58C1, mit dem residenten Plasmid pGV2260 durch ein direktes DNA-Transformationsverfahren (An, 1987) transformiert. Die Selektion rekombinanter A. *tumefaciens* Klone erfolgte unter Verwendung des Antibiotikums Kanamycin (50 mg/l).
Die Transformation der Zuckerrüben erfolgte nach Lindsey et al. (1991) unter Verwendung des Antibiotikums Kanamycin. Die Transgenität der Pflanzen wurde durch PCR überprüft. Die Verwendung der Primer GTGGAGAGGCTATTCGGTA und CCACCATGATATTCGGCAAG führte zu der Amplifikation eines 553 bp großen DNA-Fragments aus dem *npt*ll-Gen. Die PCR wurde unter Verwendung von 10 ng genomischer DNA, einer Primerkonzentration von 0,2 µM bei einer Annealingtemperatur von 55 °C in einem Multicycler PTC-200 (MJ Research, Watertown, USA) durchgeführt.

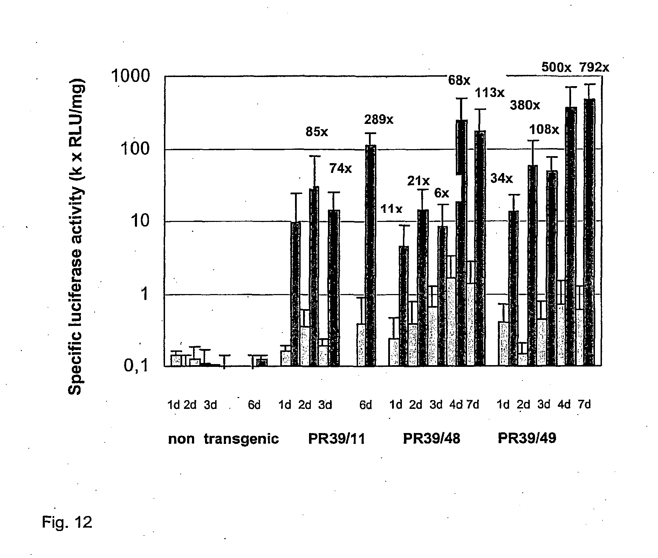

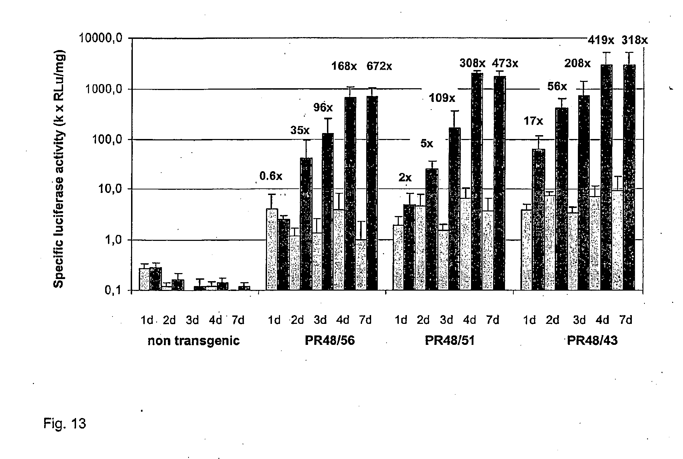

Um die Pathogeninduzerbarkeit der Promotoren zu analysieren wurden die transgenen Zuckerrüben unter *in-vitro* Bedingungen mit dem Blattfleckenerreger der Zuckerrübe, *Cercospora beticola,* infiziert. Jeweils 4 Pflanzen einer transgenen Linie wurden in eine Suspension von *C.* beticola Myzelfragmenten (400.000 Fragmente/ml) und 4 Pflanzen zu Kontrollzwecken in verdünnten Gemüsesaft getaucht. Infizierte Pflanzen und Kontrollpflanzen wurden anschließend bei 25°C und 16h Beleuchtung in einem Kulturschrank inkubiert. Infiziertes und nichtinfiziertes Blattmaterial wurde 1, 2, 3, 4 und 6-7 Tage nach der Inokulation abgenommen und die Luciferasereportergennaktivität mit dem Luciferase Assay System (Promega, Mannheim, Deutschland) wie beschrieben bestimmt.
Sowohl der 2xS-2xD als auch der 2xW2-2xD Promotor zeigten eine schnelle und starke Pathogeninduzierbarkeit in der Frühpase der Infektion, unterschieden sich jedoch in der Grundaktivität und Promotorstärke (Fig. 12-13). Der 2xS-2xD- Promotor wurde im Fall der transgenen Linien PR39/11, PR39/48 und PR39/49 sehr schnell bereits am 1. Tag nach Inokulation 11-59 fach und am 2. Tag 21-380 fach im Vergleich zu den nichtinfizierten Pflanzen induziert (Fig. 12). Während der 1. Tag noch von einem Auswachsen der Pilzhyphen auf der Epidermis gekennzeichnet ist, kommt es am 2. Tag zu einer Penetration der Blätter durch die Stomata und damit zu einem Eindringen in das Blattgewebe. In der Spätphase der Infektion am Tag 7 wurde bei einer sichtbaren Nekrosenentwicklung eine 113-792 fache Induktion des Promotors festgestellt.. Die Grundaktivität des 2xS-2xD Promotors gemessen als Reportergenaktivität der nichtinfizierten Pflanzen ist sehr gering und beträgt nur das 1-10 fache der in nichttransgenen Pflanzen messbaren Luciferaseaktivität.
Die Aktivierung des 2xW2-2xD Promotors verläuft etwas langsamer als die des 2xS-2xD Promotors. Am ersten Infektionstag weist der 2xW2-2xD Promotor nur eine 2-17 fache und am zweiten Infektionstag eine 5-56 fache Pathogeninduktion auf. Mit Auftreten der Nekrosen am 7. Tag wird eine maximale 318-672 fache Pathogeninduktion erreicht (Fig. 13). Die Grundaktivität des 2xW2-2-D Promotors ist mit dem 10-50 fachen der in nichttransgenen Pflanzen messbaren Reportergenaktivität höher als im Fall des 2xS-2xD Promotors. Deutlich hebt sich der 2xW2-2xD Promotor jedoch durch seine ca. 10-fach höhere Promotorstärke von dem 2xS-2xD Promotor ab.

### Optimierung der Promotoreigenschaften durch Veränderung der cis-Element-Anzahl

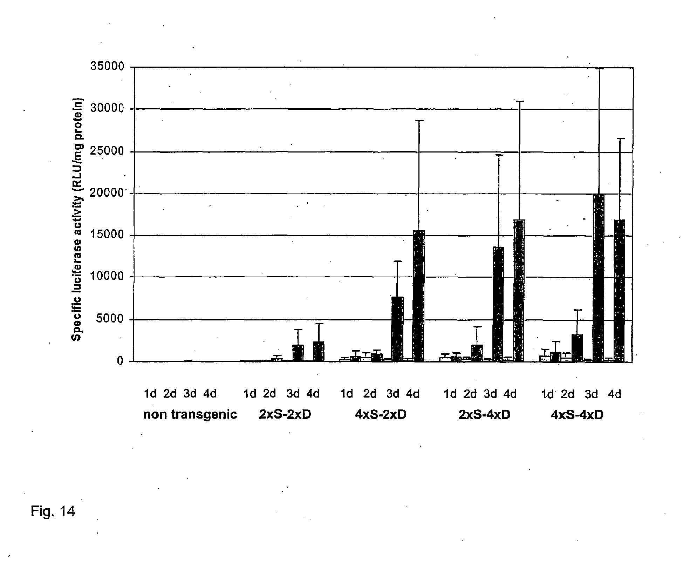

Die Eigenschaften eines synthetischen Promotors vom Typ nxS-mxD, nxW2-mxD und nxGst1-mxD mit n=1,2,3,4,5,6,7,8,9,10 und m=1,2,3,4,5,6,7,8, 9,10 lassen sich durch Variation der Anzahl der cis-Elemente entsprechend den Bedürfnissen der Genexpression modulieren und optimieren. Dies ist beispielhaft für den Promotortyp nxS-mxD gezeigt. Neben dem binären Vektor 2xS-2xD-luc-kan wurden die binären Vektoren 4xS-2xD-luc-kan, 2xS-4xD-luc-kan und 4xS-4xD-luc-kan erstellt und in Zuckerüben transformiert. Die transgenen Pflanzen wurden wie beschrieben mit *C*. *beticola* infiziert und die Reportergenaktiviät täglich nach der Pilzinokulation gemessen.
Die Messergebnisse von 13 unabhängigen 2xS-2xD-luc Linien, 14 unabhängigen 4xS-2xD-luc Linien, 15 unabhängigen 2xS-4xD-luc Linien sowie 15 unabhängigen 4xS-4xD-luc Linien wurden gemittelt und die Mittelwerte der Promotorstärke, Pathogeninduktion und Grundaktivität verglichen.
Der Vergleich der 2xS-2xD Promotoreigenschaften mir den Varianten 2xS-4xD, 4xS-2xD und 4xS-4xD zeigt, dass die durchschnittliche Promotorstärke durch die Verwendung von Tetrameren im Vergleich zu dem aus Dimeren aufgebauten Promotor erhöht wird (Fig. 14). Zudem steigt die Pathogeninduzierbarkeit vom Dimer-Dimer Promotor (2xS-2xD) über die Tetramer-Dimer und Dimer-Tetramer Promotoren (4xS-2xD, 4xS-2xD) zum Tetramer-Tetramer Promotor (4xS-4xD) zu allen Messzeitpunkten an (Tabelle 1).

**Tabelle 1: Pathogeninduzierbarkeit der Promotoren 2xS-2xD, 4xS-2xD, 2xS-4xD und 4xS-4xD in transgenen Zuckerrüben nach Infektion mit Cercospora beficola. Dargestellt ist der Mittelwert der Pathogeninduktion von 13-15 unabhängigen Transformanten (Linien) pro Promotorkonstrukt 1-4 Tage nach der Inokulation.**

| Promotor (Zahl der unabhängigen Transformanten) | 1. Tag | 2. Tag | 3. Tag | 4. Tag |
|---|---|---|---|---|
| 2xS-2xD (13 Linien) | 1,9 | 3,6 | 27 | 59 |
| 4xS-2xD (14 Linien) | 3.1 | 4,8 | 52 | 135 |
| 2xS-4xD (15 Linien) | 1,4 | 9,2 | 54 | 87 |
| 4xS-4xD (15 Linien) | 2,9 | 9,8 | 90 | 93 |

Parallel mit der Steigerung der Promotorstärke und der Pathogeninduzierbarkeit kommt es zu einer Erhöhung der Grundaktivität der Promotoren, die Tetramere enthalten (Tabelle 2).

**Tabelle 2: Grundaktivität der Promotoren 2xS-2xD, 4xS-2xD, 2xS-4xD und 4xS-4xD in Blättern transgener Zuckerrüben. Dargestellt ist der Mittelwert der Grundaktivität von 13-15 unabhängigen Transformanten (Linien) pro Promotorkonstrukt, die in dem viertägigen Infektionsversuch als nichtinfizierte Kontrollen gemessen wurden. Die Grundaktivität gibt das Verhältnis der Reportergenaktivität der transgenen Pflanzen im Vergleich zu der unspezifischen Hintergrundaktivität nichttransgener Pflanzen an.**

| Promotor (Zahl der unabhängigen Transformanten) | 1. Tag. | 2. Tag | 3. Tag | 4. Tag |
|---|---|---|---|---|
| 2xS-2xD (13 Linien) | 4,7 | 5,5 | 5,2 | 2,6 |
| 4xS-2xD (14 Linien) | 14,2 | 21 | 7,8 | 11 |
| 2xS-4xD (15 Linien) | 24,6 | 13,3 | 7 | 22,3 |
| 4xS-4xD (15 Linien) | 35,5 | 20,3 | 6,3 | 20 |

Dieses Beispiel zeigt, dass die im Zusammenhang mit dem Konzept wichtigen Promotoreigenschaften wie Promotorstärke, Pathogeninduzierbarkeit und Grundaktivität durch die Anzahl der cis-Elemente reguliert werden und für die jeweilige technische Umsetzung optimale Promotorvarianten geschaffen werden können. Die optimale Zahl an cis-Elementen von pathogeninduzierbaren Promotoren ist im untersuchten Beispiel im Hinblick auf die Pathogeninduzierbarkeit größer als die von Rushton et al., 2002 beschriebene Dimerlösung.

### Erstellung pilzresistenter Zuckerrüben durch Transformation der pathogeninduzierbaren Resistenzgen

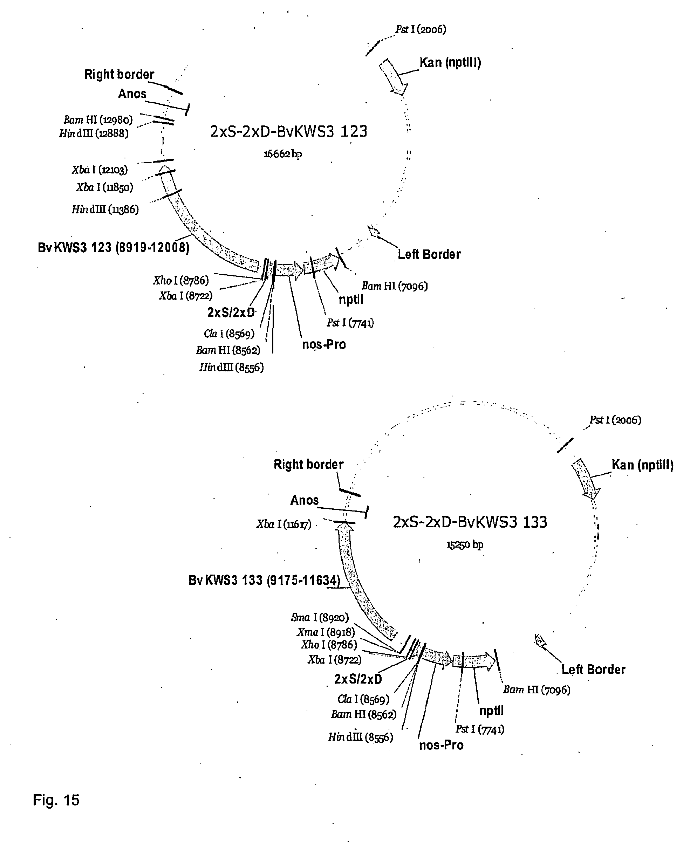

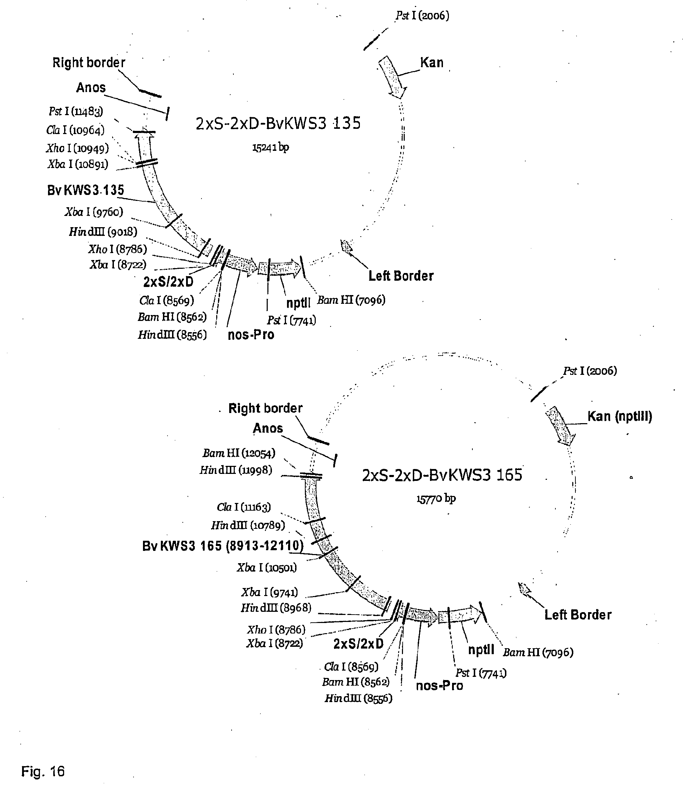

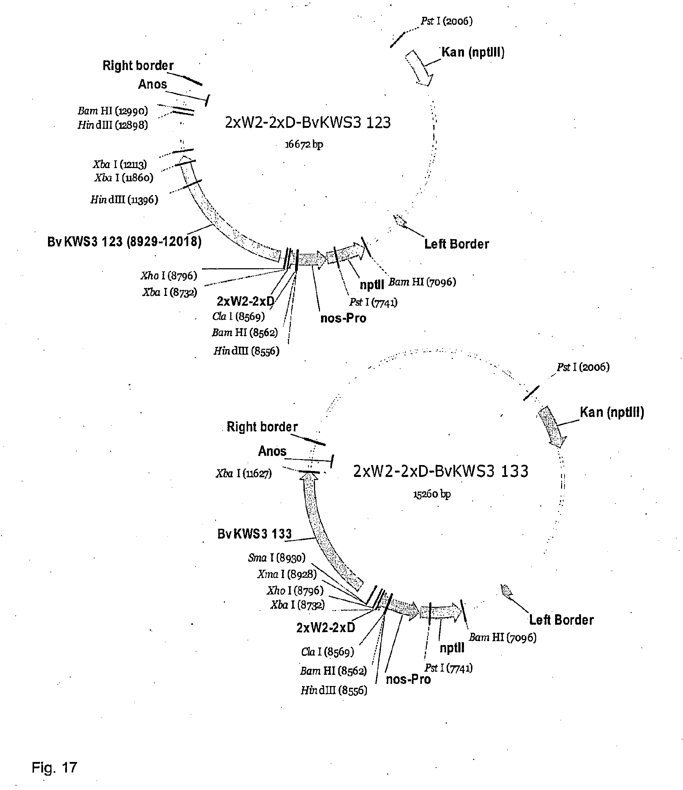

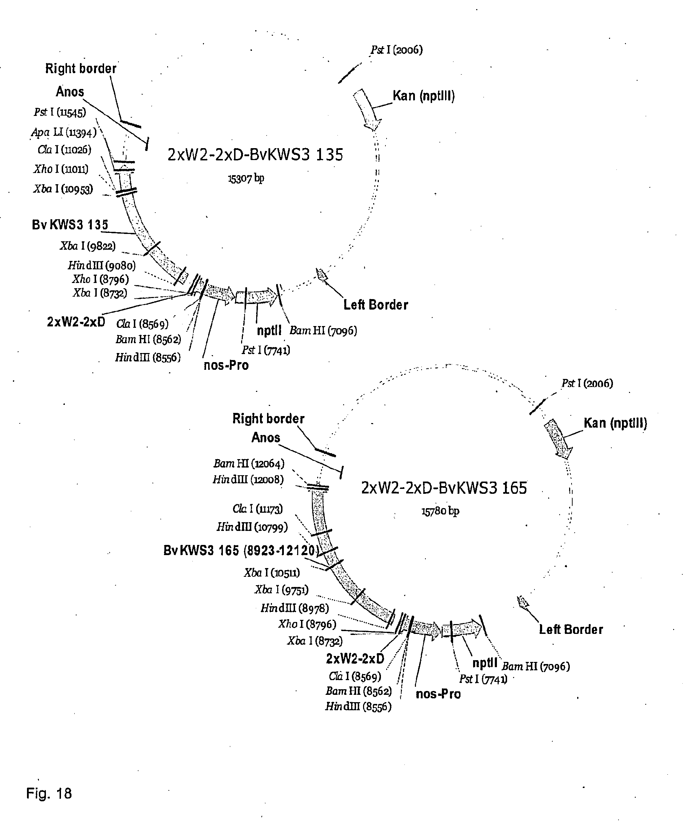

Zur Steigerung der Pilzresistenz von Zuckerrüben wurden die Promotoren 2xS-2xD bzw. 2xW₂-2xD jeweils mit einem der vier R-Gene BvKWS3_123, BvKWS3_133, BvKWS3_135 und BvKWS3_165 kombiniert und in Zuckerrüben transformiert.
Dazu wurden die 13.959 bzw. 13.969 kb großen binären Vektoren 2xS-2xD-luc-kan und 2xW2-2xD-luc-kan mit SacI geschnitten und die Schnittstelle durch T4-DNA Polymerase Behandlung aufgefüllt. Anschließend wurden die Vektoren mit Xhol nachgeschnitten, gelelektrophoretisch aufgetrennt und die 12.284 bzw. 12.294 kb großen Vektoren von dem 1.675 kb großen Lucifeasegen getrennt und isoliert.
Die Isolierung der ZR Resistenzgene erfolgt aus den Vektoren p70S-BvKWS3_123, p70S-BvKWS3_133, p70S-BvKWS3_135 und p70S-BvKWS3_165. Dazu werden die Vektoren zunächst mit Notl lineärisiert und die Schnittstelle durch Klenowbehandlung aufgefüllt. Die Vektoren werden dann mit Xhol geschnitten und die R-Gene isoliert. Die resultierenden Vektoren tragen die Bezeichnung 2xS-2xD-BvKWS3_123, 2xS-2xD-BvKWS3_133. 2xS-2xD-BvKWS3_135 und 2xS-2xD-BvKWS3_165 bzw. 2xW₂-2xD-BvKWS3_123, 2xW₂-2xD-BvKWS3_133, 2xW₂-D-BvKWS3_135 und 2xW₂-2xD-BvKWS3_165 (Fig. 15-18). Die binären Vektoren werden wie beschrieben für die Erstellung transgener Zuckerrüben verwendet.

### Identifizierung pilzresistenter Zuckerrüben durch Resistenzprüfung mit dem Schadpilz Cercospora beticola

Die erhöhte Pilzresistenz der Pflanzen wurde bei Pilzresistenzprüfungen, wie sie nachfolgend beispielhaft für die Resistenzprüfung der Zuckerrüben gegenüber *Cercospora beticola* beschrieben wird, beobachtet.
Für die Infektion von Zuckerrüben mit dem Blattfleckenerreger *C*. *beticola* werden neben den transgenen Pflanzen Zuckerrüben des für die Transformation verwendeten Genotyps 3DC4156 im Gewächshaus angezogen. Zwei Wochen vor der geplanten Inokulation werden Gemüsesaftplatten (40% Albani-Gemüsesaft) mit dem aggresiven *C. beticola* Isolat Ahlburg beimpft und bei 25 °C inkubiert. Unmittelbar vor der Inokulation wird der pilzbewachsene Agar mit Hilfe eines Objektträgers und etwas Wasser abgekratzt. Die Konzentration an Myzelfragmenten und Pilzsporen wird mit Hilfe einer Zählkammer bestimmt. Die Inokulumdichte wird durch Verdünnen mit Wasser auf eine Konzentration von 20.000 Fragmente/ml eingestellt. Für die Infektion werden die 10-12 Wochen alten Pflanzen kopfüber in ein mif dem Inokulum gefülltes 5 I Becherglas getaucht. Pro zu untersuchender Linie werden 30 Pflanzen inokuliert und die Pflanzen randomisiert im Gewächshaus aufgestellt.
Die Pflanzen werden nach der Inokulation für 4 Tage bei 28°C und 95% Luftfeuchtigkeit in einem Gewächshaus inkubiert. Nach dem vierten Tag wird die Luftfeuchtigkeit auf 60%-70% reduziert. Zwei, drei und vier Wochen nach der Inokulation wird der Befall der Blätter optisch mit Hilfe des neunteiligen Kleinwanzlebener Saatzucht (KWS) Boniturschemas (1970) bewertet (1 = gesunde Blätter, 9 = 100% zerstörte Blätter). Transgene Linien, die mit einem der Konstrukte 2xS-2xD-BvKWS3_123, 2xS-2xD-BvKWS3_133. 2xS-2xD-BvKWS3_165, 2xW₂-2xD-BvKWS3_123, 2xW₂-2xD-BvKWS3_133, 2xW2-2xD=BvKWS3_135 oder 2xW₂-2xD-BvKWS3_165 transformiert wurden, zeigten im Vergleich'zu der Kontrolle eine erhöhte Pilzresistenz (Tabelle 3).

**Tabelle 3: Resistenzsteigerung von transgenen Zuckerrüben gegenüber dem Schadpilz Cercospora beticola.**

| Kontrolle (nicht transgen) | | Transgene Linie | | | Konstrukt |
|---|---|---|---|---|---|
| T3¹ | AUDPC² | Linienbezeichnung | T3¹ | AUDPC² | |
| 6.0 | 220 | PR68-6 | 4.6 | 169 | 2xS-2xD-BvKWS3-133 |
| 6.8 | 193 | PR74-73 | 6.1 | 157 | 2xS-2xD-BvKWS3-123 |
| 4.1 | 167 | PR75-8 | 2.8 | 132 | 2xS-2xD-BvKWS3-165 |
| 6.0 | 220 | PR69-15 | 5.3 | 177 | 2xW₂-2xD-BvKWS3-133 |
| 7.0 | 226 | PR70-32 | 5.5 | 182 | 2xW₂-2xD-BvKWS3-123 |
| 6.8 | 193 | PR77-42 | 5.6 | 155 | 2xW₂-2xD-BvKWS3-135 |
| 6.8 | 229 | PR71-41 | 5.6 | 182 | 2xW₂-2xD-BvKWS3-165 |

| | | | | | |
|---|---|---|---|---|---|
| ¹Dritter und letzter Boniturwert der Resistenzprüfung (1 = gesund, 9 = 100% zerstörte Blattfläche). ²AUDPC (Area under disease progress curve) Wert ermittelt über 3 Boniturtermine (T1-T3). Der AUDPC Wert erfasst den Verlauf der Befallsstärke von mehreren Boniturzeitpunkten in einem Wert. | | | | | |

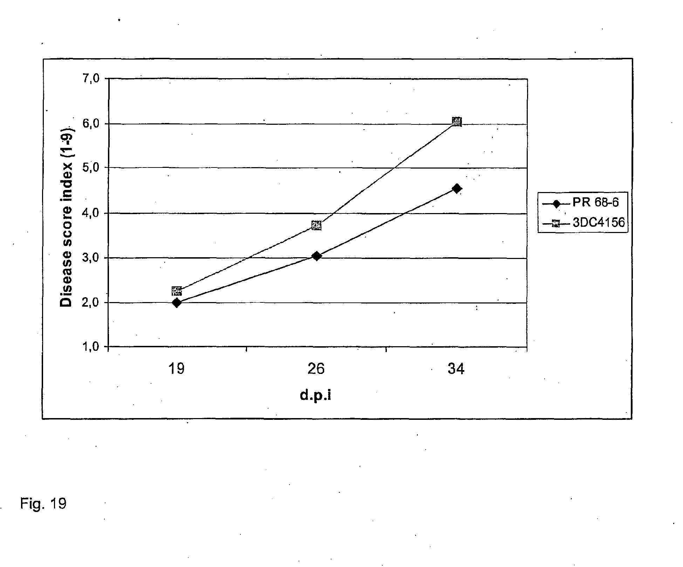

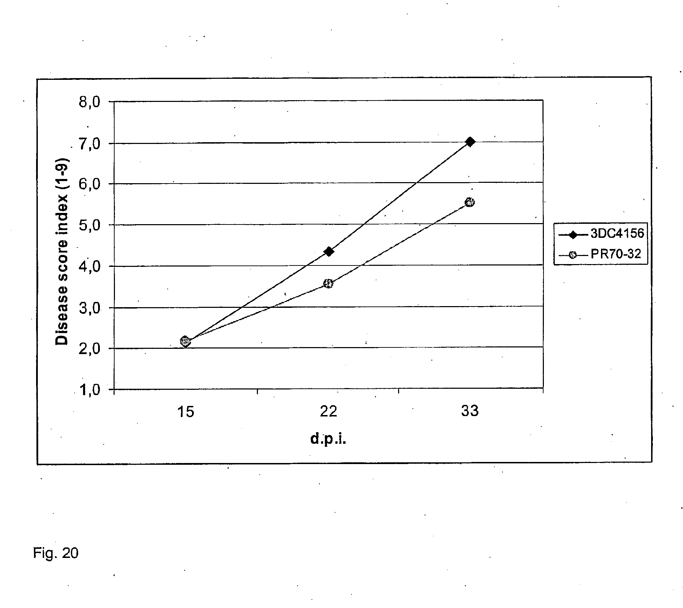

Die Analyse des zeitlichen Verlaufs der Befallsentwicklung bei der Transformante PR68-6 und PR70-32 über die drei Boniturtermine zeigt, dass mit fortschreitender Versuchsdauer die Differenz in der Befallsentwicklung zwischen Kontrolle und transgener Linie zunimmt (Fig. 19 und 20). Diese Ergebnisse zeigen, dass die induzierte Expression verschiedener R-Gene der Zuckerrübe mit Hilfe der pathogen-spezifischen Promotoren zu einer erhöhten Pilzresistenz führt.

### Erstellung pilzresistenter Pflanzen durch Transformation der N-terminalen Bereich der R-Gene unter der Kontrolle pathogenresponsiver Promotoren

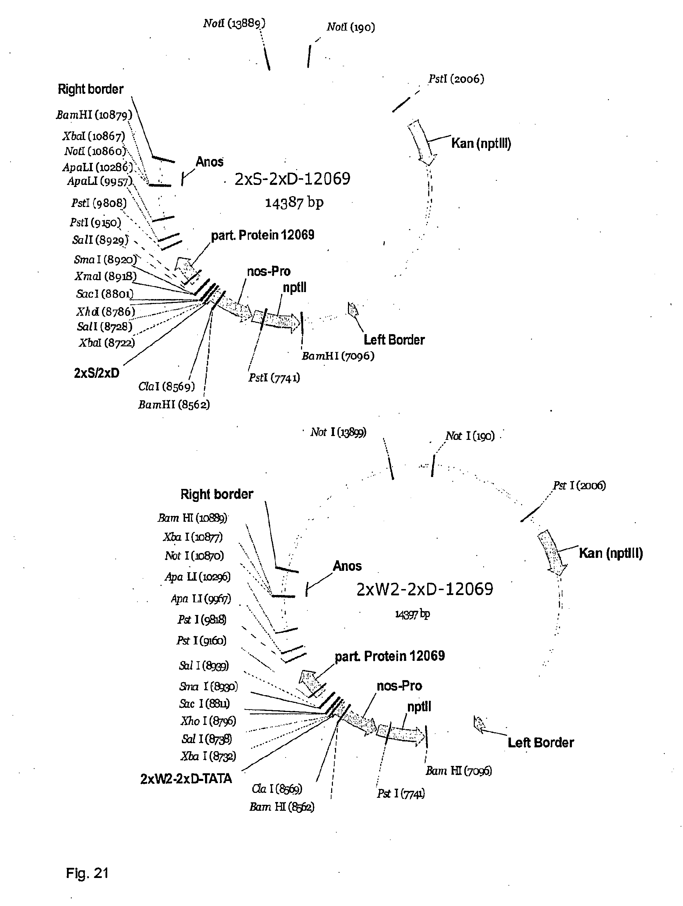

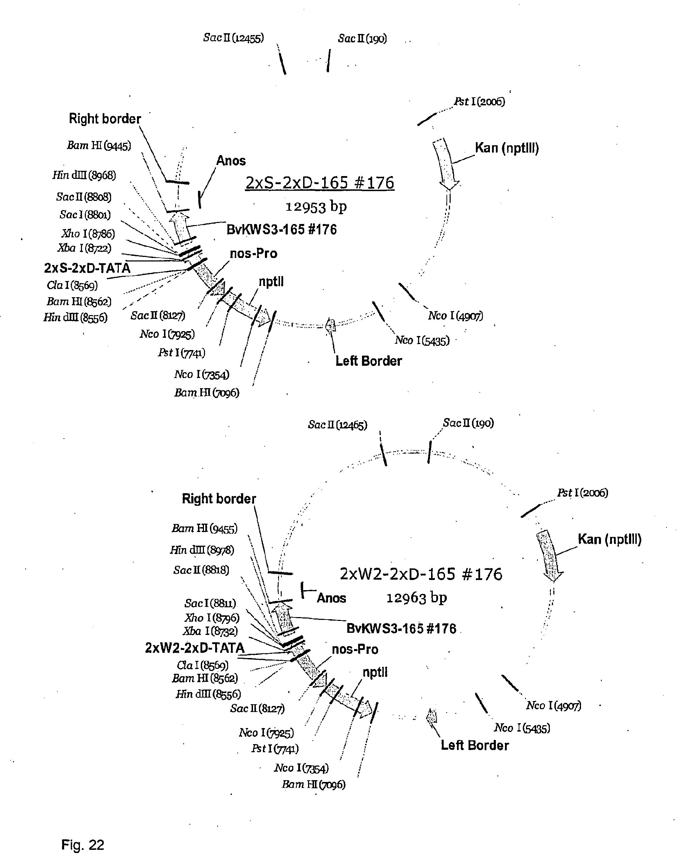

Um pilzresistentere Pflanzen unter Verwendung der N-terminalen Abschnitte der R-Gene zu erstellen, wurden die verkürzten R-Gene 13033_#159, 135_#147, 165_#175 und Bv12069 mit den Promotoren 2xS-2xD und 2xW2-2xD kombiniert und in Zuckerrüben transformiert. Dazu wurden die 13.959 bzw. 13.969 kb großen binären Vektoren 2xS-2xD-luc-kan und 2xW2-2xD-luc-kan mit SacI geschnitten und die Schnittstelle durch T4-DNA Polymerase Behandlung aufgefüllt. Anschließend wurden die Vektoren mit Xhol nachgeschnitten, gelelektrophoretisch aufgetrennt und die 12.284 bzw. 12.294 kb großen Vektoren von dem 1.675 kb großen Lucifeasegen getrennt und isoliert.
Die Isolierung der verkürzten R-Gene erfolgte aus den Vektoren p70S-12069, p70S-13033_#159, p70S-135_#147 und p70S-165_#175. Die Vektoren wurden zunächst mit Xbal linearisiert, die DNA Enden durch Klenow Behandlung aufgefüllt und die Vektoren dann mit XhoI nachgeschnitten. Die isolierten R-Genfragmente wurden dann in die vorbereiteten binären Vektoren kloniert. Die entstandenen Vektoren erhielten die Bezeichnung 2xS-2xD-12069, 2xS-2xD-13033_#159, 2xS-2xD-135_#147, 2xS-2xD-165_#175 bzw. 2xW2-2xD-12069; 2xW2-2xD-13033_#159, 2xW2-2xD-135_#147, 2xW2-2x-165_#175 (Fig. 21-22). Die binären Vektoren wurden wie beschrieben in Zuckerrüben transformiert und pilzresistentere Pflanzen durch eine *Cercospora beticola* Resistenzprüfung identifiziert.

### Referenz:

Altschul, S.F. et al. (1990). Basic Local Alignment search tool, J.Mol. Biol. 215: 403-410
An, G. (1987). Binary Ti vectors for plant transformation and promoter analysis. Methods Enzymol. 153, 292-305.
Bairoch et al., (1996) The PROSITE database, its status in 1995. Nucleic Acids Res 24:189-96
Ballavora A., Ercolana M R., Weiss J., Meksem K., Bormann C.A.I, Oberhagemann P., Salamini F., Gebhardt C. (2002). The R1 gene for potato resistance to late blight (Phytophthora infestans) belongs to the leucine zipper/NBS/LRR class of plant resistance genes. Plant J. 30(3):361-71.
Bendahmane A., Farnham G., Moffett P., and Baulcombe D.C. (2002). Constitutive gainof-function mutants in a nucleotide binding site-leucine rich repeat protein encoded at the Rx locus of potato. Plant J. Oct;32(2):195-204..
Frost D., Way H., Howles P., Luck J., Manners J., Hardham A., Finnegan J., and Ellis J. (2004). Tobacco transgenic for the flax rust resistance gene L expresses allele-specific activation of defense responses. Mol Plant Microbe Interact. 17(2):224-32.
Hennig, J., Dewey. R.E., Cutt, J. R, and Klessig, D. F. (1993). Pathogen, salicylic acid and developmental dependent expression of a beta-1,3-glucanase/GUS gene fusion in transgenic tobacco plants. Plant J. 4(3):481-93.
Howles P., Lawrence G., Finnegan J., McFadden H., Ayliffe M., Dodds D., and Ellis J. (2005). Autoactive Alleles of the Flax L6 Rust Resistance Gene Induce Non-Race-Specific Rust Resistance Associated with the Hypersensitive Response. Mol Plant Microbe Interact. 18(6):570-582.
Huang S., an der Vossen E.A., Kuang H., Vlesshouwers V.G., Zhang N:, Borm, T.J., van Eck H.J., Baker B., Jacobsen E., and Visser R.G. (2005). Comparative genomics enabled the isolation of the R3a late blight resistance gene in potato. Plant J. 42(2):251-61.
Lindsey, K,. Gallois, P., and Eady, C. (1991). Regeneration and transformation of sugar beet by Agrobacterium tumefaciens. Plant Tissue Culture Manual B7: 1-13; Kluwer Academic Publishers.
Lupas, A., Van Dyke M., and Stock J. (1991) Predicting coiled coils from protein sequences. Science 252:1162-4.
Martin, G. B., Bogdanove, A. J., and Sessa, G. (2003). Understanding the functions of plant disease resistance proteins. Annu. Rev. Plant-Biol. 54: 23-61.
Martini, N., Egen, M., Rüntz, I., and Strittmatter, G. (1993). Promoter sequences of a potato pathogenesis-related gene mediate transcriptional activation selectively upon fungal infection. Mol Gen Genet 236: 179-186.
Oldroyd, G. E. D., and. Staskawicz, B. J. (1998). Genetically engineered broad-spectrum disease resistance in tomato. Proc Natl Acad Sci U S A. 95(17): 10300-10305
Rushton P. J., Reinstadler A., Lipka V., Lippok B., Somssich I.E. (2002). Synthetic plant promoters containing defined regulatory elements provide novel insights into pathogenand wound-induced signaling. Plant Cell 14(4):749-62.
Samac, D.A and Shah, D.M. (1991). Developmental and Pathogen-Induced Activation of the Arabidopsis Acidic Chitinase Promoter. Plant Cell. 3(10):1063-1072.
Schmidt K., Heberle B., Kurrasch J., Nehls R., and Stahl D. J. (2004). Suppression of phenylalanine ammonia lyase expression in sugar beet by the fungal pathogen Cercospora beticola is mediated at the core promoter of the gene. Plant Mol. Biol., 55: 835-852.
Sonnhammer, E. L., Eddy, S. R., and Durbin, R. D. (1997) Pfam: A comprehensive database of protein domain families based on seed alignments. Proteins 28:405-20.
Tang X., Xie M., Kim Y.J., Zhou J., Klessig D.F., Martin G.B. (1999). Overexpression of Pto activates defense responses and confers broad resistance. Plant Cell 11(1):15-29.
   Tian Y., Fan L., Thurau T., Jung C., and Cai D. (2004). The absence of TIR-type resistance gene analogues in the sugar beet (Beta vulgaris L.) genome. J. Mol. Evol. 58(1):40-53.
Traut, T. W. (1994). The functions and consensus motifs of nine types of peptide segments that form different types of nucleotide binding sites. Eur. J. Biochem. 222:9-19.

### Organization Applicant

Street : Grimsehlstrasse 31
City : Einbeck
State :
Country : Germany
PostalCode : D-37555
PhoneNumber : 0049-5561-311-0
FaxNumber : 0049-5561-311-322
EmailAddress : info@kws.de
<110> OrganizationName : KWS SAAT AG
   Application Project
<120> Title : Autoaktiviertes Resistenzprotein
   <130> AppFileReference : KWS SAAT AG
   <140> CurrentAppNumber :
   <141> CurrentFilingDate :
   Sequence
<213> OrganismName :
   <400> PreSequenceString : 
gaattcgagc tccaccgcgg gattctaata cgactcacta tagggcaagc agtggtatca 60
acgcagagta cgcggrgatt catactctac ttccatactt tgtaaaaaca aaaaaagata 120
aaaatggtga taggcggaga gatttttcta tcagcgtttc ttcaagtgct ctttgaaaag 180
cttgcatcag gagggataag tctattccta aaaagagaaa aaggaatagg acctaaagtt 240
attcaaagat ggaacaaaaa attgagatta atagaggcgg ttttaagtga cgccgagcaa 300
aagcagtttc acaacaatgc cgtcaaattg tggcttcgag atcttcaaga gttcgcgtac 360
gatttagagg acatcttaga cgaatttgat acagatgctc gacttaagga gttcaatgat 420
cagcctcagc cacagccaca ggaacatcaa cccaaatctt cttgttctcc tttcaataag 480
gtacaaagct gtctttcttg tggtttccca actttaaata agaaaactac aaagtattca 540
actagcattg aagagatgag cactcgcttg gaagatcttc tggaccaagt aaggtctttg 600
gctctctcaa cgcagataca ctcaagggta gagcaacatg aagaacggag gtgaggatcc 660
<212> Type : DNA
   <211> Length : 660
   SequenceName : sequence 1
   SequenceDescription :
   Sequence
<213> OrganismName :
   <400> PreSequenceString : 
gaattcgagc tccaccgcgg gattctaata cgactcacta tagggcaagc agtggtatca 60
acgcagagta cgcggggaat ttgaagtcat catcaacctc ttatcatcat ccaactccaa 120
gattctctca tcactgacag ttctggagta gaatatggtg gacgcagtgg tcactgtgtt 180
tttggaaaaa cttttcaatg tccttgttca ggagggtggt gttttacttg gtttcaaaga 240
tcggtttaag aagctacaaa atgatctcaa atacatgcaa agcttcttta aagacgccga 300
gaggctcaaa aggaaagatg agtctctaaa atgcactctg gccgacatgc gtgagctggt 360
atatgaagct gaagacatac ttgcagattg ccagcttttg tcaaatagtt ctggaaaact 420
tcttgaagac tattctccta caaaggttcc agccaaatat caaatgggga aaagaattgg 480
tgaaatcaat gaaaagatca atagcattaa aaataacatc tcagcttttc ttggcctctt 540
tcaacctgtc catggtaatg tagaagaaga gggaccaccc agatggagtt ctccctgagg 600
atcc 604
<212> Type : DNA
   <211> Length : 604
   SequenceName : sequence 2
   SequenceDescription :
   Sequence
<213> OrganismName :
   <400> PreSequenceString : 
gaattcgagc tccaccgcgg acgcgtgggc agcagccaaa cacacacttt ctctcctgat 60
catcaatcat cattatcatc tccaaaacca aaaatggaat tcataagcac aacagtctct 120
atcgctgaaa aactgaatac tgcactgcag ttatgggagt tcaaagacaa gctctttagt 180
aacttcagct acgaaaccga acttgaggat ctccaacgca ccgtcagttc cataactgcg 240
gcgctacatg tagcagagac caagctggag ctctccgatg aactacaacg tcaaatcgag 300
gagctcaagg ataccatctt tgaagcggat gatctactgg atgagcttgt cactctttct 360
caccagcagc gggttgtaga tgccgatggt agtctcctag ataaagtaag acacttcttc 420
tctagttcca acccaatctg tgtttcttac tggatgtctc gggggagtaa ggatatcaag 480
aagaaattag atgatattgc taacaataat cagtttagct tagagcttga tcatgagcct 540
atcaggaaca gaaggcctga gacttgttct tgaggatcct 580
<212> Type : DNA
   <211> Length : 580
   SequenceName : sequence 3
   SequenceDescription :
   Sequence
<213> OrganismName :
   <400> PreSequenceString : 
agtttgtaca aaaaagcagg ctggtaccgg tccggaattc ccgggatatc gtcgacccac 60
gcgtccgctt caacaaccca tctcttcaat aattgtgaaa aaccacaaat catgattaag 120
taatttctgc accacttttg catcctcagc cccttctaaa tccttccccc tgaagtgatc 180
aataatcatt agaatgtctg aagcactact gtgggctgtc acaaaaacaa cacttgcaca 240
cattgctact tttgctggtg atcttacctg cagttatgct acacaaggca ttttagcagc 300
tcaaggtgtc cgaaacgacc tcaaaaagat cgagaacaag ctgattgcta ttcgcgctgt 360
tctgcaagat gcagaaagta agcaatatga tagcgaggct gtcaagattt ggctgaaaga 420
tgtcaaaaat gttgtgtatg atatagatga cctcttggat gaagttcaca ctgatctgtt 480
acgaaagaga atcaacaaaa gccatctgct tcgacaaatc aggtactatc tttcatcctc 540
gaatccaatt ctgagtaatt tttattggag tgataagatt agagaccttg ttcaaagatt 600
tgatgacatt gctgctaata gacgagatct tggattggat ggtcatgatc caattgaagt 660
atacaacatt gagaggaatc ctttagatgc ataatcatat gtgaagaaat cagaaattat 720
tggtagagat gaagctaggt ccgaaataat tagacgctta actagtagta ctagtgttgg 780
agatgctagt aggttggttg tgcttccgat agttgggttt ggcgggattg ggaaaacagc 840
attggcaaag cttgttttta atgatgagtg ggttgctagt aactttgata ttaagttgtg 900
ggcatgtgtc tctgaaaaat ttgatctgca gaagactaca gaagaaattt tgaattctgt 960
aagtaatgaa accactgcaa acctcaacat gagacagttg catgaaaaac tccgtgagat 1020
attggatgat cggaagcatt tccttgtgct ggacaatgtg tggatcgagg acatcaaagt 1080
gcactccaca ggaagcattt ccttgtactg gcatttagag agggtgaaga aacaagatac 1140
ccaaagcttg ctacaatcgg gaaaagcatt gttgaaaatt gtggcggggt tcctctggtt 1200
gttaaggttc ttgcaagcct gttacacagt gaaaggaacg agaaagaatg gaggcgaatt 1260
tctgaaacaa acaacataat gatatcaaac aagctttgag agtgagctac aataaactcc 1320
catctcaatt aaaagcatgt ttttcctatt gttcattatt tgtgaaggat agcaagctta 1380
atcctccgat tttgtcatgc ctgtggagtg cacttggaat cttgcaacga ggtaacgaca 1440
atgaagagtt ataatccatt ggatacaagt attgtgaaga cctatagtca agatgtcttc 1500
ttcaggatgc atttcttgtg ttcaccgaaa ttataagtga atgtagaatg cacgacttat 1560
tccatgattt agcaactgac cttgttggtg aagagatagc tgttgtgacc agtaatcatc 1620
ttaatgtttc agatatgtgc aggcatttag tatggggata tgaaggaggt gaaggtttat 1680
cagataagaa tttcccaaaa gagctactta gagcaaagaa ggctcgaact ttcagatttg 1740
gctatgcaat gggctatatt agcaagtcat ttattgaaag tattatacac aacttcaggt 1800
gcttacgtgt gttggatttg cattagagta gcttcgagga gttgtcgatg tcaattggga 1860
agttagaaca tctaagatat cttgacctgt cctacaaccc aatcatcaaa tttcttccaa 1920
gtacactatg caagttgttg aatctacaat cgttgtatgt tccaaaaaaa aaaaaaaagg 1980
gcggccgctc tagagtatcc ctcgaggggc ccaagcttac gcgtacccag ctttcttgta 2040
caaagtg 2047
<212> Type : DNA
   <211> Length : 2047
   SequenceName : sequence 4
   SequenceDescription :
   Sequence
<213> OrganismName :
   <400> PreSequenceString : gattcatact ctacttccat actttgtaaa aacaaaaaa gataaaatg gtgataggcg 60 
gagagatttt tctatcagcg tttcttcaag tgctctttga aaagcttgca tcaggaggga 120
taagtctatt cctaaaaaga gaaaaaggaa taggacctaa agttattcaa agatggaaca 180
aaaaattgag attaatagag gcggttttaa gtgacgccga gcaaaagcag tttcacaaca 240
atgccgtcaa attgtggctt cgagatcttc aagagttcgc gtacgattta gaggacatct 300
tagacgaatt tgatacagat gctcgactta aggagttcaa tgatcagcct cagccacagc 360
cacaggaaca tcaacccaaa tcttcttgtt ctcctttcaa taaggtacaa agctgtcttt 420
cttgtggttt cccaacttta aataagaaaa ctacaaagta ttcaactagc attgaagaga 480
tgagcactcg cttggaagat cttctggacc aagtaaggtc tttggctctc tcaacgcaga 540
tacactcaag ggtagagcaa catgaagaac ggagggaaac atcatctatg atacgggagc 600
cagttgtata tgggagggac gatgagaaaa atcagataat ccaaagatta ttaaagacag 660
atgagccatt ttgtgaaaat tacaatgtaa ttcctattgt tggaacagga ggcattggga 720
agacgaccct cgctcaggct gtatacaatg atgagcaggt gaaggctcac tttgatgtaa 780
aagcatgggt gtgcatctcc gatgagtttg atgtcaaaca agtgacaaca agcattatca 840
cttcagccac tcgtgataca tgtaatttcg gtgttctaga tgaggcacaa gataaattaa 900
agaacttgct tgtagacaaa agattcttga tcgttcttga tgatatatgg agtgatgaat 960
atgatccttg ggaccaactt caaacccctt tttcatcagc gaagaaggga agtagagttc 1020
taatcacgac cagaatggag acggtggcaa agaacatggt taaaagacca gatcaaagcc 1080
ccatcatcaa gttaaaggtt ttatctgatg atgattgttg gcttctcttt caaaagcatg 1140
cagtcgtaga tgaagacctc attgtgatgc agaaggactt ggttgggttg ttcaaaggat 1200
tgcctttggc tgcaaaagct ctaggaggtc tcctaaggag agagcgtaag agtaattggg 1260
caaggatatt aaagagcaat atttggagtg aagaaggtgg tgttctgcca gtcttgaggc 1320
ttagttatca tcatctccca caaaacctca aacgtgcctt tgcttattgt tctatattcc 1380
cgaaagatta taaattcacg gagatgaatg ttatattaat gtggatagca gaaggcttgc 1440
tgccagagca cgacaaggag tgcaaggaag acattggtcg tgactatttt cttgatctag 1500
tatcaagatc attgtttgaa ccaaacagtc caatgtatat ggatggatct ttcattatgc 1560
atgacctaat ccatgaccta gctcaatggg ctgctggtga agtttgttgc acattgaata 1620
tccaaaaact ttcttctaga acacgttatt ggtctttctc tgaagaaata ctgatagatc 1680
aaacatggac ttcaaagaaa cttgtccagg tacgatcatt tgcttctttt ggtgcattag 1740
atgttcgaat tcctatgcaa ctactagatt ccattttccg tcagtttcaa tacttacgct 1800
tgttgcgtat gcataaggcg ggaataattg agttaccaaa tagtatgggt aatctgaaac 1860
acttgagact tcttgacctc tcatggaata caaaactaac tagattgcca aagtccacta 1920
gcaagctttg caacctccag acgctgctgt taagaggatg taagtctctt gtggagatag 1980
tccctctaat cgaacttcaa caccttgatg ttagtagtag gtatgctgct tcaccgttgg 2040
ggattggaaa attaacaaac ttgcagacat tgaaggattt tactttgaga agatactcta 2100
agacaagtat aagtgaattg aagaacttga aatgcctccg tggctcactg gacatccatg 2160
gcctagagaa tgtgtttagt agtgaagaag cacaagcagc aaggttgcac gaaaagacag 2220
gccttgataa gttggaaatg tattggggag ataatgccca tgatgttgat gacaacatta 2280
aaaaagatgt agttgatcga ttacagcctc cggaatccat caaagaatta acgttgaatg 2340
ggtacgacgg cttaacattc cctgcttggt tgggaaatcc ctcttacact aatatggttt 2400
tcatagaatt aagggagtgt aggagatgtg aattcttacc agcactaggg cagctgccct 2460
cattaaagaa catcataata gaagggatgg atgatattaa gactgtaggc cctgaatttt 2520
acggcaatga tgatggttgt tcaaacctgt ttcctgcatt gaaaagtcta cgattctatg 2580
gaatgggggg ttgggaggag tggttggccc catcagttga caatagcaat gcatttccct 2640
gccttgaata tattagcata tatgaatgtc cattgttgcg ggctaactta ccttcccatc 2700
tcccatccct aaaagacttg tccattggca aatgcaaaga gttgagattg tcacttccaa 2760
gctgcccttt gctccaaaaa ttgactatta taacatatga gacgccattt agtattgctg 2820
attgtttgcc ctcaactctt gagaccctgg aaatcttgca ctccgaaata gagcagccaa 2880
ttcaagagtg gaacttgacc tcccttactt ctcttaaatc tcttgatctc caaaacatag 2940
gcagagccgc agatacaata gagcatattc cccaaccaga ttttcatctt ccctcttcct 3000
tgtcttcctt atatatctcg gatttcaaga atttgaaatc cttgtcatgc tctacccttc 3060
ccaacctcac tgaaattaaa atctggaatt gtaagaagct ggaatcattg ggtgtcgatt 3120
tcccaccatc gaagcttcag gaagcgtatt ttagtgattg ccctttgatt tatcaacgat 3180
gcaaaccgga tccccatgga cttattgttt ttaattatat tgataaatac aaatattcgt 3240
acatttgaag attccagttt tcattccctg aagattgagt tggaggtgga gttttggggt 3300
tttttcaga ttttgttttt gtttttttc atttctttta gtttatttgt atgaaactag 3360
aatatattca aggaattgtg ataaaaaaaa aaaaaaaagg gcggccgctc tagagtatcc 3420
ctcgaggggc ccaagcttac gcgacccagc tttcttacaa agg 3463
<212>Type : DNA
   <211> Length : 3463
   SequenceName : sequence 5
   SequenceDescription :
   Sequence
<213> OrganismName :
   <400> PreSequenceString :
   cagccaccaa agaggaccca gaat 24
   <212> Type : DNA
   <211> Length : 24
   SequenceName : sequence 6
   SequenceDescription : .
   Sequence
<213> OrganismName :
   <400> PreSequenceString :
   ttattcagcc atcaaagttg accaataat 29
   <212> Type : DNA
   <211> Length : 29
   SequenceName : sequence 7
   SequenceDescription :
   Sequence
<213> OrganismName :
   <400> PreSequenceString :
   tacaattcaa acattgttca aacaaggaac c 31
   <212> Type : DNA
   <211> Length : 31
   SequenceName : : sequence 8
   SequenceDescription :
   Sequence
<213> OrganismName :
   <400> PreSequenceString :
   ttctagccac cagatttgac caaac 25
   <212> Type : DNA
   <211> Length : 25
   SequenceName : sequence 9
   SequenceDescription :
   Sequence
<213> OrganismName :
   <400> PreSequenceString : 
actagtcagc caccaaagag gacccagaat tctagtcagc caccaaagag gacccagaat 60
tctagttaca attcaaacat tgttcaaaca aggaacctct agttacaatt caaacattgt 120
tcaaacaagg aacctctaga g 141
<212> Type : DNA
   <211> Length : 141
   SequenceName : sequence 10
   SequenceDescription :
   Sequence
<213> OrganismName :
   <400> PreSequenceString : 
actagtttat tcagccatca aagttgacca ataattctag tttattcagc catcaaagtt 60
gaccaataat tctagttaca attcaaacat tgttcaaaca aggaacctct agttacaatt 120
caaacattgt tcaaacaagg aacctctaga 150
<212> Type : DNA
   <211> Length : 150
   SequenceName : sequence 11
   SequenceDescription :
   Sequence
<213> OrganismName :
   <400> PreSequenceString : 
actagtttct agccaccaga tttgaccaaa ctctagtttc tagccaccag atttgaccaa 60
actctagtta caattcaaac attgttcaaa caaggaacct ctagttacaa ttcaaacatt 120
gttcaaacaa ggaacctcta ga 142
<212> type DNA
   <211> Length : 142
   SequenceName : sequence 12
   SequenceDescription :
   Sequence
<213> OrganismName :
   <400> PreSequenceString ;
   AVLXDAEXKQ-XXXXXLXXWL XDLKDXVYDX DDILDE 36
   <212> Type : PRT
   <211> Length : 36
   SequenceName : sequence 13
   sequenceDescription :
   Sequence
<213> OrganismName :
   <400> PreSequenceString :
   IXEIXXKLDD I 11
   <212> Type : PRT
   <211> Length : 11
   SequenceName : sequence 14
   SequenceDescription :
   Sequence
<213> OrganismName :
   <400> PreSequenceString : 
AVLXDAEXKQ XXXXXLXXWL XDLKDXVYDX DDILDEXXXX XXXXXXXXXX XXXXXXXXXX 60
XXXXXXXXXX XXXXXXXXXX XXXXXXXXIX EIXXKLDDI 99
<212> Type : PRT
   <211> Length : 99
   SequenceName : sequence 15
   SequenceDescription :
   Sequence
<213> OrganismName :
   <400> PreSequenceString : 
atggagattg gcttagcagt tggtggtgca tttctctctt cagctttgaa tgttctcttt 60
gataggcttg ctcctcacgg tgatctgctc aacatgtttc agaagcataa ggatcatgtt 120
aagctcttaa agaagctgga ggacattttg ctcggtcttc agattgtgct aagtgatgca 180
gagaataaac aagcatcaaa tcgacatgtg agccagtggt tcaataagct tcagaatgct 240
gtggacggtg ctgagaactt gatagaacaa gtcaattatg aagctttgag gcttaaggtg 300
gaaggccagc atcaaaatct tgcagaaaca agcaaccagc aagtaagtga ccttaacctg 360
tgcttcagtg atgatttctt tcttaacata aaggataagt tggaagaaac cattgaaaca 420
ttggaggtgt tggaaaagca aattggtcgc cttgtaatca ctagctag 468
<212> Typ : DNA
   <211> Length : 468
   SequenceName : sequence 16
   SequenceDescription :

## Patentansprüche

1. Autoaktiviertes Resistenzprotein zur Erzeugung einer Resistenz gegenüber Pathogenen bei Pflanzen, **dadurch gekennzeichnet, dass** das Resistenzprotein von einer Nukleinsäure codiert wird, die einen begrenzten Teil eines NBS-LRR-Resistenzgens aufweist, der sich vom 5'-Ende des codierenden Bereichs des NBS-LRR-Resistenzgens stromabwärts bis zum Beginn der NBS-Domäne des NBS-LRR-Resistenzgens erstreckt und keinen P-Loop umfasst, wobei das NBS-LRR-Resistenzgen kein TIR-NBS-LRR-Resistenzgen ist.

2. Autoaktiviertes Resistenzprotein nach Anspruch 1, das eine Aminosäuresequenz mit einem Sequenzmotiv DAE aufweist.

3. Autoaktiviertes Resistenzprotein nach Anspruch 1, das eine Aminosäuresequenz mit einem Sequenzmotiv AVLXDAE aufweist.

4. Autoaktiviertes Resistenzprotein nach Anspruch 1, **dadurch gekennzeichnet, dass** die Nukleinsäure eine Nukleotidsequenz aus folgender Gruppe aufweist:
a) Nukleotidsequenz gemäß SEQ ID NO: 1 oder eine zu der Nukleotidsequenz gemäß SEQ ID NO: 1 komplementäre Nukleotidsequenz oder eine Nukleotidsequenz, die mit der Nukleotidsequenz gemäß SEQ ID NO: 1 oder einer zu der Nukleotidsequenz gemäß SEQ ID NO: 1 komplementären Nukleotidsequenz unter stringenten Bedingungen hybridisiert;
b) Nukleotidsequenz gemäß SEQ ID NO: 2 oder eine zu der Nukleotidsequenz gemäß SEQ ID NO: 2 komplementäre Nukleotidsequenz oder eine Nukleotidsequenz, die mit der Nukleotidsequenz gemäß SEQ ID NO: 2 oder einer zu der Nukleotidsequenz gemäß SEQ ID NO: 2 komplementären Nukleotidsequenz unter stringenten Bedingungen hybridisiert;
c) Nukleotidsequenz gemäß SEQ ID NO: 3 oder eine zu der Nukleotidsequenz gemäß SEQ ID NO: 3 komplementäre Nukleotidsequenz oder eine Nukleotidsequenz, die mit der Nukleotidsequenz gemäß SEQ ID NO: 3 oder einer zu der Nukleotidsequenz gemäß SEQ ID NO: 3 komplementären Nukleotidsequenz unter stringenten Bedingungen hybridisiert;
d) Nukleotidsequenz gemäß SEQ ID NO: 4 oder eine zu der Nukleotidsequenz gemäß SEQ ID NO: 4 komplementäre Nukleotidsequenz oder eine Nukleotidsequenz, die mit der Nukleotidsequenz gemäß SEQ ID NO: 4 oder einer zu der Nukleotidsequenz gemäß SEQ ID NO: 4 komplementären Nukleotidsequenz unter stringenten Bedingungen hybridisiert;
e) Nukleotidsequenz gemäß SEQ ID NO: 16 oder eine zu der Nukleotidsequenz gemäß SEQ ID NO: 16 komplementäre Nukleotidsequenz oder eine Nukleotidsequenz, die mit der Nukleotidsequenz gemäß SEQ ID NO: 16 oder einer zu der Nukleotidsequenz gemäß SEQ ID NO: 16 komplementären Nukleotidsequenz unter stringenten Bedingungen hybridisiert.

5. Autoaktiviertes Resistenzprotein nach Anspruch 1, **dadurch gekennzeichnet, dass** es sich bei dem NBS-LRR-Resistenzgen um ein Resistenzgen aus Zuckerrübe oder Kartoffel handelt.

6. Autoaktiviertes Resistenzprotein nach Anspruch 1, das eine Aminosäuresequenz mit einer Sequenz aus folgender Gruppe aufweist:
a) SEQ ID NO: 13
b) SEQ ID NO: 14
c) SEQ ID NO: 15

7. Transgene Pflanze mit einem autoaktivierten Resistenzprotein nach einem der vorhergehenden Ansprüche.

8. Teile einer transgenen Pflanze nach Anspruch 7.

9. Transgene Samen oder transgenes Saatgut mit einem autoaktivierten Resistenzprotein nach einem der Ansprüche 1 bis 6.

10. Verfahren zur Herstellung einer transgenen Pflanze mit gesteigerter Resistenz gegenüber Pathogenen sowie von transgenen Samen oder transgenem Saatgut von einer solchen Pflanze unter Verwendung einer Nukleinsäure, wobei die Nukleinsäure für ein autoaktiviertes Resistenzprotein codiert und einen begrenzten Teil eines NBS-LRR-Resistenzgens aufweist, der sich vom 5'-Ende des codierenden Bereichs des NBS-LRR-Resistenzgens stromabwärts bis zum Beginn der NBS-Domäne des NBS-LRR-Resistenzgens erstreckt und keinen P-Loop umfasst, und wobei das NBS-LRR-Resistenzgen kein TIR-NBS-LRR-Resistenzgen ist.

11. Verfahren nach Anspruch 10, **dadurch gekennzeichnet, dass** die Nukleinsäure für eine Aminosäuresequenz mit einem Sequenzmotiv DAE codiert.

12. Verfahren nach Anspruch 10, **dadurch gekennzeichnet, dass** die Nukleinsäure für eine Aminosäuresequenz mit einem Sequenzmotiv AVLXDAE codiert.

13. Verfahren nach Anspruch 10, **dadurch gekennzeichnet, dass** die Nukleinsäure eine Nukleotidsequenz aus folgender Gruppe aufweist:
a) Nukleotidsequenz gemäß SEQ ID NO: 1 oder eine zu der Nukleotidsequenz gemäß SEQ ID NO: 1 komplementäre Nukleotidsequenz oder eine Nukleotidsequenz, die mit der Nukleotidsequenz gemäß SEQ ID NO: 1 oder einer zu der Nukleotidsequenz gemäß SEQ ID NO: 1 komplementären Nukleotidsequenz unter stringenten Bedingungen hybridisiert;
b) Nukleotidsequenz gemäß SEQ ID NO: 2 oder eine zu der Nukleotidsequenz gemäß SEQ ID NO: 2 komplementäre Nukleotidsequenz oder eine Nukleotidsequenz, die mit der Nukleotidsequenz gemäß SEQ ID NO: 2 oder einer zu der Nukleotidsequenz gemäß SEQ ID NO: 2 komplementären Nukleotidsequenz unter stringenten Bedingungen hybridisiert;
c) Nukleotidsequenz gemäß SEQ ID NO: 3 oder eine zu der Nukleotidsequenz gemäß SEQ ID NO: 3 komplementäre Nukleotidsequenz oder eine Nukleotidsequenz, die mit der Nukleotidsequenz gemäß SEQ ID NO: 3 oder einer zu der Nukleotidsequenz gemäß SEQ ID NO: 3 komplementären Nukleotidsequenz unter stringenten Bedingungen hybridisiert;
d) Nukleotidsequenz gemäß SEQ ID NO: 4 oder eine zu der Nukleotidsequenz gemäß SEQ ID NO: 4 komplementäre Nukleotidsequenz oder eine Nukleotidsequenz, die mit der Nukleotidsequenz gemäß SEQ ID NO: 4 oder einer zu der Nukleotidsequenz gemäß SEQ ID NO: 4 komplementären Nukleotidsequenz unter stringenten Bedingungen hybridisiert,
e) Nukleotidsequenz gemäß SEQ ID NO: 16 oder eine zu der Nukleotidsequenz gemäß SEQ ID NO: 16 komplementäre Nukleotidsequenz oder eine Nukleotidsequenz, die mit der Nukleotidsequenz gemäß SEQ ID NO: 16 oder einer zu der Nukleotidsequenz gemäß SEQ ID NO: 16 komplementären Nukleotidsequenz unter stringenten Bedingungen hybridisiert.

14. Verfahren nach Anspruch 10, **dadurch gekennzeichnet, dass** es sich bei dem NBS-LRR-Resistenzgen um ein Resistenzgen aus Zuckerrübe oder Kartoffel handelt.

15. Verfahren nach Anspruch 10, **dadurch gekennzeichnet, dass** die Nukleinsäure für eine Aminosäuresequenz mit einer Sequenz ausgewählt aus folgender Gruppe codiert:
a) SEQ ID NO: 13
b) SEQ ID NO: 14
c) SEQ ID NO: 15

16. Verfahren nach Anspruch 10, **dadurch gekennzeichnet, dass** man ein Nukleinsäurekonstrukt mit
a) einem pathogeninduzierbaren Promotor sowie
b) einer unter der Kontrolle des Promotors stehenden Nukleinsäure gemäß einem der Ansprüche 10 bis 15 verwendet.

17. Verfahren nach Anspruch 16, **dadurch gekennzeichnet, dass** es sich bei dem pathogeninduzierbaren Promotor um einen synthetischen Promotor handelt.

18. Verfahren nach Anspruch 17, **dadurch gekennzeichnet, dass** der synthetische Promotor eines oder mehrere der folgenden cis-Elementkombinationen umfasst, wobei n und m eine natürliche Zahl von 1...10 bedeuten:
a) eine nxS-mxD-Box
b) eine nxW2-mxD-Box
c) eine nxGst1-mxD-Box

19. Verfahren nach Anspruch 18, **dadurch gekennzeichnet, dass** die cis-Elementkombination
a) eine Nukleotidsequenz der SEQ ID NO: 10 oder
b) eine Nukleotidsequenz der SEQ ID NO: 11 oder
c) eine Nukleotidsequenz der SEQ ID NO: 12 umfasst.

## Claims

1. Autoactivated resistance protein for producing a resistance against pathogens in plants, thereby **characterized** that the resistance protein is encoded by a nucleic acid comprising a limited part of a NBS-LRR-resistance gene, which extends from the 5' end of the coding region of the NBS-LRR-resistance gene downstream to the beginning of the NBS-domain of the NBS-LRR-resistance gene, but not including a P-loop, wherein the NBS-LRR-resistance gene is not a TIR-NBS-LRR-resistance gene.

2. Autoactivated resistance protein according to claim 1, comprising an amino acid sequence with the sequence motif DAE.

3. Autoactivated resistance protein according to claim 1, comprising an amino acid sequence with the sequence motif AVLXDAE.

4. Autoactivated resistance protein according to claim 1, thereby **characterized** that the nucleic acid comprises a nucleotide sequence from the following group:
a) nucleotide sequence according to SEQ ID NO: 1 or a nucleotide sequence complementary to the nucleotide sequence according to SEQ ID NO: 1 or a nucleotide sequence which hybridizes with the nucleotide sequence according to SEQ ID NO: 1 or the nucleotide sequence complementary to the nucleotide sequence according to SEQ ID NO: 1 under stringent conditions.
b) nucleotide sequence according to SEQ ID NO: 2 or a nucleotide sequence complementary to the nucleotide sequence according to SEQ ID NO: 2 or a nucleotide sequence which hybridizes with the nucleotide sequence according to SEQ ID NO: 2 or the nucleotide sequence complementary to the nucleotide sequence according to SEQ ID NO: 2 under stringent conditions.
c) nucleotide sequence according to SEQ ID NO: 3 or a nucleotide sequence complementary to the nucleotide sequence according to SEQ ID NO: 3 or a nucleotide sequence which hybridizes with the nucleotide sequence according to SEQ ID NO: 3 or the nucleotide sequence complementary to the nucleotide sequence according to SEQ ID NO: 3 under stringent conditions.
d) nucleotide sequence according to SEQ ID NO: 4 or a nucleotide sequence complementary to the nucleotide sequence according to SEQ ID NO: 4 or a nucleotide sequence which hybridizes with the nucleotide sequence according to SEQ ID NO: 4 or the nucleotide sequence complementary to the nucleotide sequence according to SEQ ID NO: 4 under stringent conditions.
e) nucleotide sequence according to SEQ ID NO: 16 or a nucleotide sequence complementary to the nucleotide sequence according to SEQ ID NO: 16 or a nucleotide sequence which hybridizes with the nucleotide sequence according to SEQ ID NO: 16 or the nucleotide sequence complementary to the nucleotide sequence according to SEQ ID NO: 16 under stringent conditions.

5. Autoactivated resistance protein according to claim 1, thereby **characterized** that the NBS-LRR-resistance gene is a resistance gene from sugar beet or potato.

6. Autoactivated resistance protein according to claim 1, comprising an amino acid sequence with a seqeuence selected from the following group:
a) SEQ ID NO: 13
b) SEQ ID NO: 14
c) SEQ ID NO: 15

7. Transgenic plant with an autoactivated resistance protein according to one of the preceding claims.

8. Parts of a transgenic plant according to claim 7.

9. Transgenic seed or transgenic seed material with an autoactivated resistance protein according to one of claims 1 to 6.

10. Process for producing a transgenic plant with an increased resistance against pathogens as well as transgenic seed or transgenic seed material of such a plant using a nucleic acid, wherein the nucleic acid encodes an autoacivated resistance protein and comprises a limited part of a NBS-LRR-resistance gene, which extends from the 5' end of the coding region of the NBS-LRR-resistance gene downstream to the beginning of the NBS-domain of the NBS-LRR-resistance gene, but not including a P-loop, wherein the NBS-LRR-resistance gene is not a TIR-NBS-LRR-resistance gene.

11. Process according to claim 10, thereby **characterized** that the nucleic acid codes for an amino acid sequence with the sequence motif DAE.

12. Process according to claim 10, thereby **characterized** that the nucleic acid codes for an amino acid sequence with the sequence motif AVLXDAE.

13. Process according to claim 10, thereby **characterized** that the nucleic acid comprises a nucleotide sequence from the following group:
a) nucleotide sequence according to SEQ ID NO: 1 or a nucleotide sequence complementary to the nucleotide sequence according to SEQ ID NO: 1 or a nucleotide sequence, which hybridizes with the nucleotide sequence according to SEQ ID NO: 1 or the nucleotide sequence complementary to the nucleotide sequence according to SEQ ID NO: 1 under stringent conditions.
b) nucleotide sequence according to SEQ ID NO: 2 or a nucleotide sequence complementary to the nucleotide sequence according to SEQ ID NO: 2 or a nucleotide sequence, which hybridizes with the nucleotide sequence according to SEQ ID NO: 2 or the nucleotide sequence complementary to the nucleotide sequence according to SEQ ID NO: 2 under stringent conditions.
c) nucleotide sequence according to SEQ ID NO: 3 or a nucleotide sequence complementary to the nucleotide sequence according to SEQ ID NO: 3 or a nucleotide sequence, which hybridizes with the nucleotide sequence according to SEQ ID NO: 3 or the nucleotide sequence complementary to the nucleotide sequence according to SEQ ID NO: 3 under stringent conditions..
d) nucleotide sequence according to SEQ ID NO: 4 or a nucleotide sequence complementary to the nucleotide sequence according to SEQ ID NO: 4 or a nucleotide sequence, which hybridizes with the nucleotide sequence according to SEQ ID NO: 4 or the nucleotide sequence complementary to the nucleotide sequence according to SEQ ID NO: 4 under stringent conditions.
e) nucleotide sequence according to SEQ ID NO: 16 or a nucleotide sequence complementary to the nucleotide sequence according to SEQ ID NO: 16 or a nucleotide sequence, which hybridizes with the nucleotide sequence according to SEQ ID NO: 16 or the nucleotide sequence complementary to the nucleotide sequence according to SEQ ID NO: 16 under stringent conditions.

14. Process according to claim 10, thereby **characterized** that the NBS-LRR-resistance gene is a resistance gene from sugar beet or potato.

15. Process according to claim 10, thereby **characterized** that the nucleic acid encodes an amino acid sequence with a sequence selected from the following group:
a) SEQ ID NO: 13
b) SEQ ID NO: 14
c) SEQ ID NO: 15

16. Process according to claim 10, thereby **characterized** that a nucleic acid construct is used comprising:
a) a pathogen inducible promoter as well as
b) a nucleic acid according to one of claims 10 to 15, controlled by the promoter

17. Process according to claim 16, thereby **characterized** that the pathogen inducible promoter is a synthetic promoter.

18. Process according to claim 17, thereby **characterized** that the synthetic promoter includes one or more of the following cis-element combinations, wherein n and m mean a natural number from 1...10:
a) a nxS-mxD-Box
b) a nxW2-mxD-Box
c) a nxGst1-mxD-Box

19. Process according to claim 18, thereby **characterized** that the cis-element combination includes:
a) a nucleotide sequence according to SEQ ID NO: 10 or
b) a nucleotide sequence according to SEQ ID NO: 11 or
c) a nucleotide sequence according to SEQ ID NO: 12

## Revendications

1. Protéine de résistance auto-activée, destinée à provoquer chez des plantes une résistance à des pathogènes, **caractérisée en ce que** ladite protéine de résistance est codée par un acide nucléique comportant une partie limitée d'un gène de résistance de type NBS-LRR, ladite partie s'étendant de l'extrémité 5' de la région codante du gène de résistance de type NBS-LRR jusqu'au début du domaine NBS situé en aval dudit gène de résistance de type NBS-LRR et ne présentant pas de boucle P, ledit gène de résistance de type NBS-LRR n'étant pas un gène de résistance de type TIR-NBS-LRR.

2. Protéine de résistance auto-activée selon la revendication 1, présentant une séquence d'acides aminés renfermant un motif de séquence de type DAE.

3. Protéine de résistance auto-activée selon la revendication 1, présentant une séquence d'acides aminés renfermant un motif de séquence de type AVLXDAE.

4. Protéine de résistance auto-activée selon la revendication 1, **caractérisée en ce que** ledit acide nucléique présente une séquence de nucléotides choisie dans le groupe suivait :
a) la séquence de nucléotides selon SEQ ID N° : 1 ou une séquence de nucléotides complémentaire à la séquence de nucléotides selon SEQ ID N° : 1 ou une séquence de nucléotides qui s'hybride, dans des conditions stringentes, avec la séquence de nucléotides selon SEQ ID N° : 1 ou avec une séquence de nucléotides complémentaire à la séquence de nucléotides selon SEQ ID N° : 1 ;
b) la séquence de nucléotides selon SEQ ID N° : 2 ou une séquence de nucléotides complémentaire à la séquence de nucléotides selon SEQ ID N° : 2 ou une séquence de nucléotides qui s'hybride, dans des conditions stringentes, avec la séquence de nucléotides selon SEQ ID N° : 2 ou avec une séquence de nucléotides complémentaire à la séquence de nucléotides selon SEQ ID N° : 2 ;
c) la séquence de nucléotides selon SEQ ID N° : 3 ou une séquence de nucléotides complémentaire à la séquence de nucléotides selon SEQ ID N° : 3 ou une séquence de nucléotides qui s'hybride, dans des conditions stringentes, avec la séquence de nucléotides selon SEQ ID N° : 3 ou avec une séquence de nucléotides complémentaire à la séquence de nucléotides selon SEQ ID N° : 3 ;
d) la séquence de nucléotides selon SEQ ID N° : 4 ou une séquence de nucléotides complémentaire à la séquence de nucléotides selon SEQ ID N° : 4 ou une séquence de nucléotides qui s'hybride, dans des conditions stringentes, avec la séquence de nucléotides selon SEQ ID N° : 4 ou avec une séquence de nucléotides complémentaire à la séquence de nucléotides selon SEQ ID N° : 4 ;
e) la séquence de nucléotides selon SEQ ID N° : 16 ou une séquence de nucléotides complémentaire à la séquence de nucléotides selon SEQ ID N° : 16 ou une séquence de nucléotides qui s'hybride, dans des conditions stringentes, avec la séquence de nucléotides selon SEQ ID N° : 16 ou avec une séquence de nucléotides complémentaire à la séquence de nucléotides selon SEQ ID N° : 16.

5. Protéine de résistance auto-activée selon la revendication 1, **caractérisée en ce que** ledit gène de résistance de type NBS-LRR est un gène de résistance issu de la betterave sucrière ou de la pomme de terre.

6. Protéine de résistance auto-activée selon la revendication 1, présentant une séquence d'acides aminés choisie dans le groupe suivant :
a) SEQ ID N° : 13
b) SEQ ID N° : 14
c) SEQ ID N° : 15

7. Plante transgénique avec une protéine de résistance auto-activée selon l'une des revendications précédentes.

8. Parties d'une plante transgénique selon la revendication 7.

9. Graines transgéniques ou semence transgénique avec une protéine de résistance auto-activée selon l'une des revendications 1 à 6.

10. Procédé de production d'une plante transgénique ayant une résistance accrue vis-à-vis des pathogènes ainsi que de graines transgéniques ou d'une semence transgénique issues d'une telle plante, en mettent en oeuvre un acide nucléique, ledit acide nucléique codant pour une protéine de résistance auto-activée et comportant une partie limitée d'un gène de résistance de type NBS-LRR, ladite partie s'étendant de l'extrémité 5' de la région codante du gène de résistance de type NBS-LRR jusqu'au début du domaine NBS situé en aval dudit gène de résistance de type NBS-LRR et ne présentant pas de boucle P, et ledit gène de résistance de type NBS-LRR n'étant pas un gène de résistance de type TIR-NBS-LRR.

11. Procédé selon la revendication 10, **caractérisé en ce que** ledit acide nucléique code pour une séquence d'acides aminés renfermant un motif de séquence de type DAE.

12. Procédé selon la revendication 10, **caractérisé en ce que** ledit acide nucléique code pour une séquence d'acides aminés renfermant un motif de séquence de type AVLXDAE.

13. Procédé selon la revendication 10, **caractérisé en ce que** ledit acide nucléique présente une séquence de nucléotides choisie dans le groupe suivant :
a) la séquence de nucléotides selon SEQ ID N° : 1 ou une séquence de nucléotides complémentaire à la séquence de nucléotides selon SEQ ID N° : 1 ou une séquence de nucléotides qui s'hybride, dans des conditions stringentes, avec la séquence de nucléotides selon SEQ ID N° : 1 ou avec une séquence de nucléotides complémentaire à la séquence de nucléotides selon SEQ ID N° : 1 ;
b) la séquence de nucléotides selon SEQ ID N° : 2 ou une séquence de nucléotides complémentaire à la séquence de nucléotides selon SEQ ID N° : 2 ou une séquence de nucléotides qui s'hybride, dans des conditions stringentes, avec la séquence de nucléotides selon SEQ ID N° : 2 ou avec une séquence de nucléotides complémentaire à la séquence de nucléotides selon SEQ ID N° : 2 ;
c) la séquence de nucléotides selon SEQ ID N° : 3 ou une séquence de nucléotides complémentaire à la séquence de nucléotides selon SEQ ID N° : 3 ou une séquence de nucléotides qui s'hybride, dans des conditions stringentes, avec la séquence de nucléotides selon SEQ ID N° : 3 ou avec une séquence de nucléotides complémentaire à la séquence de nucléotides selon SEQ ID N° : 3 ;
d) la séquence de nucléotides selon SEQ ID N° : 4 ou une séquence de nucléotides complémentaire à la séquence de nucléotides selon SEQ ID N° : 4 ou une séquence de nucléotides qui s'hybride, dans des conditions stringentes, avec la séquence de nucléotides selon SEQ ID N° : 4 ou avec une séquence de nucléotides complémentaire à la séquence de nucléotides selon SEQ ID N° : 4,
e) la séquence de nucléotides selon SEQ ID N° : 16 ou une séquence de nucléotides complémentaire à la séquence de nucléotides selon SEQ ID N° : 16 ou une séquence de nucléotides qui s'hybride, dans des conditions stringentes, avec la séquence de nucléotides selon SEQ ID N° : 16 ou avec une séquence de nucléotides complémentaire à la séquence de nucléotides selon SEQ ID N° : 16.

14. Procédé selon la revendication 10, **caractérisé en ce que** ledit gène de résistance de type NBS-LRR est un gène de résistance issu de la betterave sucrière ou de la pomme de terre.

15. Procédé selon la revendication 10, **caractérisé en ce que** ledit acide nucléique code pour une séquence d'acides aminés avec une séquence choisie dans le groupe suivant :
a) SEQ ID N° : 13
b) SEQ ID N° : 14
c) SEQ ID N° : 15

16. Procédé selon la revendication 10, **caractérisé en ce qu'**on utilise une construction d'acide nucléique renfermant
a) un promoteur inductible par des pathogènes et
b) un acide nucléique selon l'une des revendications 10 à 15 qui contrôlé par ledit promoteur.

17. Procédé selon la revendication 16, **caractérisé en ce que** ledit promoteur inductible par des pathogènes est un promoteur synthétique.

18. Procédé selon la revendication 17, **caractérisé en ce que** ledit promoteur synthétique comprend une ou plusieurs des combinaisons d'éléments de type cis suivantes, n et m représentant un nombre entier naturel compris entre 1 et 10 :
a) une nxS-mxD-box
b) une nxW2-mxD-box
c) une nxGst1-mxD-box

19. Procédé selon la revendication 18, **caractérisé en ce que** ladite combinaison d'éléments de type cis comprend
a) une séquence de nucléotides de la SEQ ID N° : 10 ou
b) une séquence de nucléotides de la SEQ ID N° : 11 ou
c) une séquence de nucléotides de la SEQ ID N° : 12.
